(19) Europäisches Patentamt
European Patent Office
Office européen des brevets

(11) EP 3 614 998 B1

(12) EUROPEAN PATENT SPECIFICATION

(45) Date of publication and mention of the grant of the patent:
**31.12.2025 Bulletin 2026/01**

(21) Application number: **18727910.4**

(22) Date of filing: **25.04.2018**

(51) International Patent Classification (IPC):
***A61K 8/46*** (2006.01) ***A61K 8/73*** (2006.01)
***A61K 8/81*** (2006.01) ***A61K 8/49*** (2006.01)
***A61K 8/04*** (2006.01) ***A61Q 5/00*** (2006.01)
***A61Q 5/02*** (2006.01)

(52) Cooperative Patent Classification (CPC):
**A61K 8/8147; A61K 8/046; A61K 8/463; A61K 8/4926; A61K 8/494; A61K 8/731; A61K 8/737; A61K 8/817; A61K 8/8182; A61Q 5/006; A61Q 5/02;** A61K 2800/5424; A61K 2800/5426; A61K 2800/594

(86) International application number:
**PCT/US2018/029313**

(87) International publication number:
**WO 2018/200644 (01.11.2018 Gazette 2018/44)**

(54) **HAIR CARE COMPOSITIONS WITH ANIONIC AND CATIONIC POLYMERS**

HAARPFLEGEZUSAMMENSETZUNGEN MIT ANIONISCHEN UND KATIONISCHEN POLYMEREN

COMPOSITIONS POUR LES CHEVEUX AVEC DES POLYMERES ANIONIQUES ET CATIONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2017 US 201762490301 P**

(43) Date of publication of application:
**04.03.2020 Bulletin 2020/10**

(73) Proprietor: **The Procter & Gamble Company**
**Cincinnati, OH 45202 (US)**

(72) Inventors:
- **CHANG, Debora W.**
**Cincinnati, Ohio 45202 (US)**
- **JOHNSON, Eric Scott**
**Cincinnati, Ohio 45202 (US)**
- **KROGER LYONS, Kelly Rose**
**Cincinnati, Ohio 45202 (US)**
- **FIGUEROA, Rebekah Ruth**
**Cincinnati, Ohio 45202 (US)**
- **FINLEY, Rebecca Ann**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2006/042174**
**WO-A1-2015/055432**
**WO-A1-2016/172409**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 614 998 B1

## Description

FIELD OF THE INVENTION

The present invention is directed to hair care compositions where it has been found that the addition of certain anionic polymers in combination with one or more cationic polymers provide an unexpected increase in coacervate formation as well as deposition benefits.

BACKGROUND OF THE INVENTION

For years, anti-dandruff shampoos have been widely used to treat dandruff and clean hair and scalp, but there still remains a need for improved anti-dandruff shampoos. In general, anti-dandruff shampoos are formulated with anti-dandruff agents in combination with surfactants and aqueous systems that are intended to deposit the anti-dandruff agents on the scalp. The anti-dandruff agents can be insoluble particulates such as zinc pyrithione and/or surfactant soluble substances such as climbazole or octopirox. Many anti-dandruff shampoos use cationic polymers with anionic surfactants to form coacervate which aid in the deposition of insoluble particulate agents. However, generally coacervates do not impact soluble agents deposition as the soluble agents do not associate with the coacervates formed between the cationic polymers and anionic surfactants. Indeed it can prove difficult to deposit on scalp much more than 1-2% of the soluble agents present in anti-dandruff shampoos while the remaining 98-99% of the soluble agents in the formulas are rinsed away. As many of the anti-dandruff agents can be relatively expensive, allowing >97% of the soluble agents to rinse away is equivalent to pouring money down the drain, and so there remains a need for a shampoo that can more efficiently deposit soluble anti-dandruff agents. Also, as consumers continue to desire a shampoo that delivers superior anti-dandruff efficacy and lower agent deposition results in lower anti-dandruff efficacy, there remains a need for a shampoo that can deposit on scalp a higher percentage of the soluble agents present in anti-dandruff shampoos.

The association of many classes of surfactants into micellar aggregates is a well-known phenomenon. Micelles are often drawn as static structures of spherical aggregates, but in truth micelles are in dynamic equilibrium with individual surfactant molecules (monomers) that are constantly being exchanged between the bulk and the micelles. Additionally, the micelles themselves are continuously disintegrating and reassembling. There are two relaxation processes involved in micellar solutions. The first is a fast relaxation process referred to as $\tau_1$, which is associated with the quick exchange of monomers between micelles and the surrounding bulk phase. The second relaxation time, $\tau_2$, is attributed to the micelle formation and dissolution process (i.e., the lifetime of the micelle). Extensive experimental research on the kinetics of micellization by Shah and co-workers (Patist, A., Jha, B.K., Oh, S.G., and Shah, D.O., J. Surfactants Deterg. 2, 317, (1999); James-Smith, M.A., Shekhawat, D., and Shah, D.O., Tenside Surf. Det. 44, 142 (2007)) showed a strong correlation of $\tau_2$ with a number of detergency properties including oil solubilization in micellar solutions and droplet size in emulsions, as well as surfactant properties such as dynamic surface tension and micelle stability. Their research also showed a strong inverse correlation of $\tau_2$ with other properties such as foamability and concentration of sub-micellar aggregates. Specifically, they showed that a maximal $\tau_2$ and thus maximal micellar stability corresponded to both a maximal rate of oil solubilization and maximal amount of oil solubilized. Logic would therefore suggest that a cleansing composition with longer $\tau_2$, more stable micelles, and faster rate of solubilization would be preferred since such a system can clean better, more quickly solubilize larger quantities of oils or surfactant-soluble materials and should be more stable. However it has been shown that a composition with a surfactant system of shorter $\tau_2$ and less stable micelles is preferred because that composition can deposit surfactant soluble anti-dandruff agents with significantly greater efficiency. As a consequence of the preferred compositions having less stable micelles, these compositions do not readily form elongated micelles, which are a critical component for the formation of coacervates, since upon dilution the elongated negatively charged micelles are needed to associate with cationic polymers to form coacervate. Coacervates are known to provide wet conditioning and wet detangling from a shampoo, which are consumer desired benefits. Indeed hair care compositions that contain both cationic polymers and surfactants that form less stable micelles form little to no coacervate upon dilution, unlike what is observed in typical shampoos.

WO 2016/172409 A1 refers to a hair care composition directed to from about 14% to about 40% of one or more surfactants; from about 0.1% to 10% of one or more surfactant soluble antidandruff agents; wherein when the hair care composition is diluted to 1.3% surfactant concentration, the hair care composition has a ratio of surfactant fiffusion coefficient to soluble agent diffusion coefficient less than 0.6 or greater than 1.4.

It has been surprisingly found that the addition of certain anionic polymers in combination with one or more cationic polymers provide an unexpected increase in coacervate formation as well as a deposition benefit.

SUMMARY OF THE INVENTION

A hair care composition comprising from about 10% to about 25% of one or more surfactants, by weight of the

composition, wherein the one or more surfactants is an anionic surfactant selected from the group consisting of anionic alkyl sulfates and alkyl ether sulfates having straight or branched alkyl chains and mixtures thereof, wherein the total amount of one or more surfactants ranges from 10% to 25% by weight of the composition; from about 0.01% to 10% of one or more surfactant soluble antidandruff agents, by weight of the composition, wherein the one or more surfactant soluble antidandruff agents is one material or a mixture selected from the groups consisting of: azoles being climbazole, ketoconazole, itraconazole, econazole, and elubiol; hydroxy pyridones being piroctone olamine, ciclopirox, rilopirox, and MEA-Hydroxyoctyloxypyridinone; kerolytic agent being salicylic acid; strobilurin being azoxystrobin; and metal chelator being 1,10-phenanthroline, wherein the total amount of one or more surfactant soluble antidandruff agents ranges from 0.01% to 10% by weight of the composition; from about 1% to 2.5% of one or more anionic polymers selected from the group consisting of anionic polymers with anionic functional groups, anionic polymers which are homopolymers having a monomer, or copolymers having more than one type of monomer including one or more monomers with anionic functional groups and other monomers that contain non anionic functional groups and mixtures thereof, by weight of the composition; from about 0.2% to 1% of one or more cationic polymers, by weight of the composition; wherein the composition has a deposition efficiency of greater than 1.2X that of a control composition, as measured according to the Anti-dandruff Agent Deposition Test Method as disclosed herein, wherein the control composition comprises 14% SLE1S by weight of the composition, no polymeric constituents, 1% of the surfactant soluble antidandruff agent by weight of the composition at a pH of about 6, preferably wherein the composition has a deposition efficiency of greater than or equal to 1.4X that of a control composition wherein the control composition comprises 14% SLE1S by weight of the composition, 1% of the surfactant soluble antidandruff agent by weight of the composition at a pH of 6.

## DETAILED DESCRIPTION OF EMBODIMENTS OF THE INVENTION

All percentages and ratios used herein are by weight of the total composition, unless otherwise designated. All measurements are understood to be made at ambient conditions, where "ambient conditions" means conditions at about 25°C, under about one atmosphere of pressure, and at about 50% relative humidity, unless otherwise designated. All numeric ranges are inclusive of narrower ranges; delineated upper and lower range limits are combinable to create further ranges not explicitly delineated.

The compositions of the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "application," as used in reference to a composition, means to apply or spread the compositions of the present invention onto keratinous tissue such as the hair.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

While the specification concludes with claims particularly pointing out and distinctly claiming the invention, it is believed that the present invention will be better understood from the following description.

As used herein, the term "fluid" includes liquids and gels.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "Molecular weight" refers to the weight average molecular weight unless otherwise stated. Molecular weight is measured using industry standard method, gel permeation chromatography ("GPC").

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the hair care composition.

As used herein, "personal care compositions" includes products such as shampoos, shower gels, liquid hand cleansers, hair colorants, facial cleansers, and other surfactant-based liquid compositions

As used herein, the terms "include," "includes," and "including," are meant to be non-limiting and are understood to mean "comprise," "comprises," and "comprising," respectively.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

**Soluble Anti-dandruff Agent**

Anti-dandruff agent is one material or a mixture selected from the groups consisting of: azoles, such as climbazole, ketoconazole, itraconazole, econazole, and elubiol; hydroxy pyridones, such as piroctone olamine, ciclopirox, rilopirox, and MEA-Hydroxyoctyloxypyridinone; kerolytic agents, such as salicylic acid and other hydroxy acids; strobilurins such as azoxystrobin and metal chelators such as 1,10-phenanthroline.

The azole anti-microbials may be an imidazole selected from the group consisting of: benzimidazole, benzothiazole, bifonazole, butaconazole nitrate, climbazole, clotrimazole, croconazole, eberconazole, econazole, elubiol, fenticonazole, fluconazole, flutimazole, 6 isoconazole, ketoconazole, lanoconazole, metronidazole, miconazole, neticonazole, omoconazole, oxiconazole nitrate, sertaconazole, sulconazole nitrate, tioconazole, thiazole, and mixtures thereof, or the azole anti-microbials is a triazole selected from the group consisting of: terconazole, itraconazole, and mixtures thereof. The azole anti-microbial agent may be ketoconazole. The sole anti-microbial agent may be ketoconazole.

The soluble anti-dandruff agent may be present in an amount from about 0.01% to 10%, from about 0.1% to about 9%, from about 0.25% to 8%, and from about 0.5% to 6%. The soluble antidandruff agent can be surfactant soluble and thus surfactant soluble antidandruff agents.

A. DETERSIVE SURFACTANT

The hair care composition may comprise greater than about 10% by weight of a surfactant system which provides cleaning performance to the composition; further the composition may comprise greater than 12% by weight of a surfactant system which provides cleaning performance to the composition. The surfactant system comprises an anionic surfactant and/or a combination of anionic surfactants and/or a combination of anionic surfactants and co-surfactants selected from the group consisting of amphoteric, zwitterionic, nonionic and mixtures thereof. Various examples and descriptions of detersive surfactants are set forth in U.S. Patent No. 8,440,605; U.S. Patent Application Publication No. 2009/155383; and U.S. Patent Application Publication No. 2009/0221463.

The hair care composition comprises from about 10% to about 25%, from about 10% to about 18%, from about 10% to about 14%, from about 10% to about 12%, from about 11% to about 20%, from about 12% to about 20%, and/or from about 12% to about 18% by weight of one or more surfactants.

Anionic surfactants suitable for use in the compositions are the alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278.

Exemplary anionic surfactants for use in the hair care composition include ammonium lauryl sulfate, ammonium laureth sulfate, ammonium C10-15 pareth sulfate, ammonium C10-15 alkyl sulfate, ammonium C11-15 alkyl sulfate, ammonium decyl sulfate, ammonium deceth sulfate, ammonium undecyl sulfate, ammonium undeceth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, sodium C10-15 pareth sulfate, sodium C10-15 alkyl sulfate, sodium C11-15 alkyl sulfate, sodium decyl sulfate, sodium deceth sulfate, sodium undecyl sulfate, sodium undeceth sulfate, potassium lauryl sulfate, potassium laureth sulfate, potassium C10-15 pareth sulfate, potassium C10-15 alkyl sulfate, potassium C11-15 alkyl sulfate, potassium decyl sulfate, potassium deceth sulfate, potassium undecyl sulfate, potassium undeceth sulfate, sodium lauryl sarcosinate, sodium lauroyl sarcosinate, lauryl

sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. The anionic surfactant may be sodium lauryl sulfate or sodium laureth sulfate.

The composition of the present invention can also include anionic surfactants selected from the group consisting of:

a) $R_1\ O(CH_2CHR_3O)_y\ SO_3M$;
b) $CH_3\ (CH_2)_z\ CHR_2\ CH_2\ O\ (CH_2\ CHR_3O)_y\ SO_3M$; and
c) mixtures thereof,

where $R_1$ represents $CH_3\ (CH_2)_{10}$, $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is about 1 when y is not zero (0), and M is a monovalent or divalent, positively-charged cation.

Suitable anionic alkyl sulfates and alkyl ether sulfate surfactants include, but are not limited to, those having branched alkyl chains which are synthesized from C8 to C18 branched alcohols which may be selected from the group consisting of: Guerbet alcohols, aldol condensation derived alcohols, oxo alcohols, F-Toxo alcohols and mixtures thereof. Non-limiting examples of the 2-alkyl branched alcohols include oxo alcohols such as 2-methyl-1-undecanol, 2-ethyl-1-decanol, 2-propyl-1-nonanol, 2-butyl 1-octanol, 2-methyl-1-dodecanol, 2-ethyl-1-undecanol, 2-propyl-1-decanol, 2-butyl-1-nonanol, 2-pentyl-1-octanol, 2-pentyl-1-heptanol, and those sold under the tradenames LIAL® (Sasol), ISALCHEM® (Sasol), and NEODOL® (Shell), and Guerbet and aldol condensation derived alcohols such as 2-ethyl-1-hexanol, 2-propyl-1-butanol, 2-butyl-1-octanol, 2-butyl-1-decanol, 2-pentyl-1-nonanol, 2-hexyl-1-octanol, 2-hexyl-1-decanol and those sold under the tradename ISOFOL® (Sasol) or sold as alcohol ethoxylates and alkoxylates under the tradenames LUTENSOL XP® (BASF) and LUTENSOL XL® (BASF).

The anionic alkyl sulfates and alkyl ether sulfates may also include those synthesized from C8 to C18 branched alcohols derived from butylene or propylene which are sold under the trade names EXXAL™ (Exxon) and Marlipal® (Sasol). This includes anionic surfactants of the subclass of sodium trideceth-n sulfates (STnS), where n is between about 0.5 and about 3.5. Exemplary surfactants of this subclass are sodium trideceth-2 sulfate and sodium trideceth-3 sulfate. The composition of the present invention can also include sodium tridecyl sulfate.

The composition of the present invention can also include anionic alkyl and alkyl ether sulfosuccinates and/or dialkyl and dialkyl ether sulfosuccinates and mixtures thereof. The dialkyl and dialkyl ether sulfosuccinates may be a C6-15 linear or branched dialkyl or dialkyl ether sulfosuccinate. The alkyl moieties may be symmetrical (i.e., the same alkyl moieties) or asymmetrical (i.e., different alkyl moieties). Nonlimiting examples include: disodium lauryl sulfosuccinate, disodium laureth sulfosuccinate, sodium bistridecyl sulfosuccinate, sodium dioctyl sulfosuccinate, sodium dihexyl sulfosuccinate, sodium dicyclohexyl sulfosuccinate, sodium diamyl sulfosuccinate, sodium diisobutyl sulfosuccinate, linear bis(tridecyl) sulfosuccinate and mixtures thereof.

The hair care composition may comprise a co-surfactant. The co-surfactant can be selected from the group consisting of amphoteric surfactant, zwitterionic surfactant, non-ionic surfactant and mixtures thereof. The co-surfactant can include, but is not limited to, lauramidopropyl betaine, cocoamidopropyl betaine, lauryl hydroxysultaine, sodium lauroamphoacetate, disodium cocoamphodiacetate, cocamide monoethanolamide and mixtures thereof.

The hair care composition may further comprise from about 0.25% to about 15%, from about 1% to about 14%, from about 2% to about 13% by weight of one or more amphoteric, zwitterionic, nonionic co-surfactants, or a mixture thereof.

Suitable amphoteric or zwitterionic surfactants for use in the hair care composition herein include those which are known for use in shampoo or other hair care cleansing. Non limiting examples of suitable zwitterionic or amphoteric surfactants are described in U.S. Patent Nos. 5,104,646 and 5, 106,609.

Amphoteric co-surfactants suitable for use in the composition include those surfactants described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Suitable amphoteric surfactant include, but are not limited to, thoseselected from the group consisting of: sodium cocaminopropionate, sodium cocaminodipropionate, sodium cocoamphoacetate, sodium cocoamphodiacetate, sodium cocoamphohydroxypropylsulfonate, sodium cocoamphopropionate, sodium cornamphopropionate, sodium lauraminopropionate, sodium lauroamphoacetate, sodium lauroamphodiacetate, sodium lauroamphohydroxypropylsulfonate, sodium lauroamphopropionate, sodium cornamphopropionate, sodium lauriminodipropionate, ammonium cocaminopropionate, ammonium cocaminodipropionate, ammonium cocoamphoacetate, ammonium cocoamphodiacetate, ammonium cocoamphohydroxypropylsulfonate, ammonium cocoamphopropionate, ammonium cornamphopropionate, ammonium lauraminopropionate, ammonium lauroamphoacetate, ammonium lauroamphodiacetate, ammonium lauroamphohydroxypropylsulfonate, ammonium lauroamphopropionate, ammonium cornamphopropionate, ammonium lauriminodipropionate, triethanolamine cocaminopropionate, triethanolamine cocaminodipropionate, triethanolamine cocoamphoacetate, triethanolamine cocoamphohydroxypropylsulfonate, triethanolamine cocoamphopropionate, triethanolamine cornamphopropionate, triethanolamine lauraminopropionate, triethanolamine lauroamphoacetate, triethanolamine lauroamphohydroxypropylsulfonate, triethanolamine lauroamphopropionate, triethanolamine cornamphopropionate, triethanolamine lauriminodipropionate, cocoamphodipropionic acid, disodium caproamphodiacetate, disodium caproamphoadipropionate, disodium capryloamphodiacetate, disodium capryloamphodipriopionate, disodium cocoamphocarboxyethylhydroxypropylsulfonate, disodium cocoamphodiacetate, disodium cocoamphodipropionate, disodium dicarboxyethylcocopropylenediamine, disodium laureth-5 carboxyamphodiacetate, disodium lauriminodipropionate, disodium lauroamphodiacetate, disodium 1auroamphodipropionate, disodium oleoamphodipropionate, disodium PPG-2-isodecethyl-7 carboxyamphodiacetate, lauraminopropionic acid, lauroamphodipropionic acid, lauryl aminopropylglycine, lauryl diethylenediaminoglycine, and mixtures thereof

The composition may comprises a zwitterionic co-surfactant, wherein the zwitterionic surfactant is a derivative of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. The zwitterionic surfactant can be selected from the group consisting of: cocamidoethyl betaine, cocamidopropylamine oxide, cocamidopropyl betaine, cocamidopropyl dimethylaminohydroxypropyl hydrolyzed collagen, cocamidopropyldimonium hydroxypropyl hydrolyzed collagen, cocamidopropyl hydroxysultaine, cocobetaineamido amphopropionate, coco-betaine, coco-hydroxysultaine, coco/oleamidopropyl betaine, coco-sultaine, lauramidopropyl betaine, lauryl betaine, lauryl hydroxysultaine, lauryl sultaine, and mixtures thereof.

Suitable nonionic surfactants for use in the present invention include those described in McCutcheion's Detergents and Emulsifiers, North American edition (1986), Allured Publishing Corp., and McCutcheion's Functional Materials, North American edition (1992). Suitable nonionic surfactants for use in the personal care compositions of the present invention include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl glycosides, alkyl polyglucosides, alkylamine oxides, and polyoxyethylenated silicones.

The co-surfactant can be a non-ionic surfactant selected from the alkanolamides group including: Cocamide, Cocamide Methyl MEA, Cocamide DEA, Cocamide MEA, Cocamide MIPA, Lauramide DEA, Lauramide MEA, Lauramide MIPA, Myristamide DEA, Myristamide MEA, PEG-20 Cocamide MEA, PEG-2 Cocamide, PEG-3 Cocamide, PEG-4 Cocamide, PEG-5 Cocamide, PEG-6 Cocamide, PEG-7 Cocamide, PEG-3 Lauramide, PEG-5 Lauramide, PEG-3 Oleamide, PPG-2 Cocamide, PPG-2 Hydroxyethyl Cocamide, PPG-2 Hydroxyethyl Isostearamide and mixtures thereof.

Representative polyoxyethylenated alcohols include alkyl chains ranging in the C9-C16 range and having from about 1 to about 110 alkoxy groups including, but not limited to, laureth-3, laureth-23, ceteth-10, steareth-10, steareth-100, beheneth-10, and commercially available from Shell Chemicals, Houston, Texas under the trade names Neodol® 91, Neodol® 23, Neodol® 25, Neodol® 45, Neodol® 135, Neodo®l 67, Neodol® PC 100, Neodol® PC 200, Neodol® PC 600, and mixtures thereof.

Also available commercially are the polyoxyethylene fatty ethers available commercially under the Brij® trade name from Uniqema, Wilmington, Delaware, including, but not limited to, Brij® 30, Brij® 35, Brij® 52, Brij® 56, Brij® 58, Brij® 72, Brij® 76, Brij® 78, Brij® 93, Brij® 97, Brij® 98, Brij® 721 and mixtures thereof.

Suitable alkyl glycosides and alkyl polyglucosides can be represented by the formula (S)nO-R wherein S is a sugar moiety such as glucose, fructose, mannose, galactose, and the like; n is an integer of from about 1 to about 1000, and R is a C8-C30 alkyl group. Examples of long chain alcohols from which the alkyl group can be derived include decyl alcohol, lauryl alcohol, myristyl alcohol, cetyl alcohol, stearyl alcohol, oleyl alcohol, and the like. Examples of these surfactants include alkyl polyglucosides wherein S is a glucose moiety, R is a C8-20 alkyl group, and n is an integer of from about 1 to about 9. Commercially available examples of these surfactants include decyl polyglucoside and lauryl polyglucoside available under trade names APG® 325 CS, APG® 600 CS and APG® 625 CS) from Cognis, Ambler, Pa. Also useful herein are sucrose ester surfactants such as sucrose cocoate and sucrose laurate and alkyl polyglucosides available under trade names Triton™ BG-10 and Triton™ CG-110 from The Dow Chemical Company, Houston, Tx.

Other nonionic surfactants suitable for use in the present invention are glyceryl esters and polyglyceryl esters, including but not limited to, glyceryl monoesters, glyceryl monoesters of C12-22 saturated, unsaturated and branched chain fatty acids such as glyceryl oleate, glyceryl monostearate, glyceryl monopalmitate, glyceryl monobehenate, and mixtures thereof, and polyglyceryl esters of C12-22 saturated, unsaturated and branched chain fatty acids, such as polyglyceryl-4 isostearate, polyglyceryl-3 oleate, polyglyceryl-2- sesquioleate, triglyceryl diisostearate, diglyceryl monooleate, tetraglyceryl monooleate, and mixtures thereof.

Also useful herein as nonionic surfactants are sorbitan esters. Sorbitan esters of C12-22 saturated, unsaturated,

and branched chain fatty acids are useful herein. These sorbitan esters usually comprise mixtures of mono-, di-, tri-, etc. esters. Representative examples of suitable sorbitan esters include sorbitan monolaurate (SPAN® 20), sorbitan monopalmitate (SPAN® 40), sorbitan monostearate (SPAN® 60), sorbitan tristearate (SPAN® 65), sorbitan monooleate (SPAN® 80), sorbitan trioleate (SPAN® 85), and sorbitan isostearate.

Also suitable for use herein are alkoxylated derivatives of sorbitan esters including, but not limited to, polyoxyethylene (20) sorbitan monolaurate (Tween® 20), polyoxyethylene (20) sorbitan monopalmitate (Tween® 40), polyoxyethylene (20) sorbitan monostearate (Tween® 60), polyoxyethylene (20) sorbitan monooleate (Tween® 80), polyoxyethylene (4) sorbitan monolaurate (Tween® 21), polyoxyethylene (4) sorbitan monostearate (Tween® 61), polyoxyethylene (5) sorbitan monooleate (Tween® 81), and mixtures thereof, all available from Uniqema.

Also suitable for use herein are alkylphenol ethoxylates including, but not limited to, nonylphenol ethoxylates (Tergitol™ NP-4, NP-6, NP-7, NP-8, NP-9, NP-10, NP-11, NP-12, NP-13, NP-15, NP-30, NP-40, NP-50, NP-55, NP-70 available from The Dow Chemical Company, Houston, Tx.) and octylphenol ethoxylates (Triton™ X-15, X-35, X-45, X-114, X-100, X-102, X-165, X-305, X-405, X-705 available from The Dow Chemical Company, Houston, Tx).

Also suitable for use herein are tertiary alkylamine oxides including lauramine oxide and cocamine oxide.

Non limiting examples of other anionic, zwitterionic, amphoteric, and non-ionic additional surfactants suitable for use in the hair care composition are described in McCutcheon's, Emulsifiers and Detergents, 1989 Annual, published by M. C. Publishing Co., and U.S. Patent Nos. 3,929,678, 2,658,072; 2,438,091; 2, 528, 378.

Suitable surfactant combinations comprise an average weight % of alkyl branching of from about 0.5% to about 30%, alternatively from about 1% to about 25%, alternatively from about 2% to about 20%. The surfactant combination can have a cumulative average weight % of C8 to C12 alkyl chain lengths of from about 7.5% to about 25%, alternatively from about 10% to about 22.5%, alternatively from about 10% to about 20%.The surfactant combination can have an average C8-C12 / C13-C18 alkyl chain ratio from about 3 to about 200, alternatively from about 25 to about 175.5, alternatively from about 50 to about 150, alternatively from about 75 to about 125.

B. CATIONIC POLYMERS

The hair care composition also comprises a cationic polymer. These cationic polymers can include at least one of (a) a cationic guar polymer, (b) a cationic non-guar galactomannan polymer, (c) a cationic tapioca polymer, (d) a cationic copolymer of acrylamide monomers and cationic monomers, and/or (e) a synthetic, non-crosslinked, cationic polymer, which may or may not form lyotropic liquid crystals upon combination with the detersive surfactant (f) a cationic cellulose polymer. Additionally, the cationic polymer can be a mixture of cationic polymers.

The hair care composition may comprise a cationic guar polymer, which is a cationically substituted galactomannan (guar) gum derivatives. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of β(1-4) glycosidic linkages. The galactose branching arises by way of an α(1-6) linkage. Cationic derivatives of the guar gums are obtained by reaction between the hydroxyl groups of the polygalactomannan and reactive quaternary ammonium compounds. The degree of substitution of the cationic groups onto the guar structure should be sufficient to provide the requisite cationic charge density described above.

The cationic polymer, may include but not limited to a cationic guar polymer, has a weight average Molecular weight of less than 2.2 million g/mol, or from about 150 thousand to about 2.2 million g/mol, or from about 200 thousand to about 2.2 million g/mol, or from about 300 thousand to about 1.2 million g/mol, or from about 750,000 thousand to about 1 million g/mol. The cationic guar polymer may have a charge density of from about 0.2 to about 2.2 meq/g, or from about 0.3 to about 2.0 meq/g, or from about 0.4 to about 1.8 meq/g; or from about 0.5 meq/g to about 1.8 meq/g.

The cationic guar polymer may have a weight average Molecular weight of less than about 1.5 million g/mol, and has a charge density of from about 0.1 meq/g to about 2.5 meq/g. The cationic guar polymer may have a weight average molecular weight of less than 900 thousand g/mol, or from about 150 thousand to about 800 thousand g/mol, or from about 200 thousand to about 700 thousand g/mol, or from about 300 thousand to about 700 thousand g/mol, or from about 400 thousand to about 600 thousand g/mol. from about 150 thousand to about 800 thousand g/mol, or from about 200 thousand to about 700 thousand g/mol, or from about 300 thousand to about 700 thousand g/mol, or from about 400 thousand to about 600 thousand g/mol. The cationic guar polymer may have a charge density of from about 0.2 to about 2.2 meq/g, or from about 0.3 to about 2.0 meq/g, or from about 0.4 to about 1.8 meq/g; or from about 0.5 meq/g to about 1.5 meq/g.

The cationic guar polymer may be formed from quaternary ammonium compounds. The quaternary ammonium compounds for forming the cationic guar polymer may conform to the general formula 1:

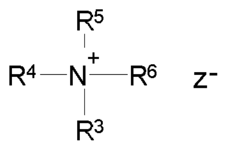

wherein where $R^3$, $R^4$ and $R^5$ are methyl or ethyl groups; $R^6$ is either an epoxyalkyl group of the general formula 2:

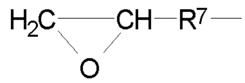

or $R^6$ is a halohydrin group of the general formula 3:

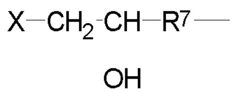

wherein $R^7$ is a $C_1$ to $C_3$ alkylene; X is chlorine or bromine, and Z is an anion such as Cl-, Br-, I- or $HSO_4$-.

The cationic guar polymer may conform to the general formula 4:

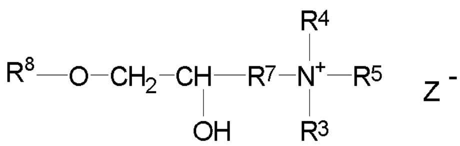

wherein $R^8$ is guar gum; and wherein $R^4$, $R^5$, $R^6$ and $R^7$ are as defined above; and wherein Z is a halogen. The cationic guar polymer may conform to Formula 5:

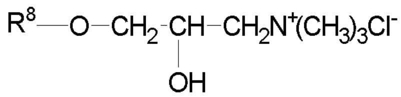

Suitable cationic guar polymers include cationic guar gum derivatives, such as guar hydroxypropyltrimonium chloride. The cationic guar polymer may be a guar hydroxypropyltrimonium chloride. Specific examples of guar hydroxypropyltrimonium chlorides include the Jaguar® series commercially available from Solvay, for example Jaguar® C-500, commercially available from Solvay. Jaguar® C-500 has a charge density of 0.8 meq/g and a molecular weight of 500,000 g/mol. Other suitable guar hydroxypropyltrimonium chloride are: guar hydroxypropyltrimonium chloride which has a charge density of about 1.3 meq/g and a molecular weight of about 500,000 g/mol and is available from Solvay as Jaguar® Optima. Other suitable guar hydroxypropyltrimonium chloride are: guar hydroxypropyltrimonium chloride which has a charge density of about 0.7 meq/g and a molecular weight of about 1,500,000 g/mol and is available from Solvay as Jaguar® Excel. Other suitable guar hydroxypropyltrimonium chloride are: guar hydroxypropyltrimonium chloride which has a charge density of about 1.1 meq/g and a molecular weight of about 500,000 g/mol and is available from ASI, a charge density of about 1.5 meq/g and a molecular weight of about 500,000 g/mole is available from ASI.

Other suitable guar hydroxypropyltrimonium chloride are: Hi-Care 1000, which has a charge density of about 0.7 meq/g and a Molecular weight of about 600,000 g/mole and is available from Solvay; N-Hance 3269 and N-Hance 3270, which have a charge density of about 0.7 meq/g and a molecular weight of about 425,000 g/mol and are available from ASI; N-Hance 3196, which has a charge density of about 0.8 meq/g and a molecular weight of about 1,100,000 g/ mol and is available from ASI. AquaCat CG518 has a charge density of about 0.9 meq/g and a Molecular weight of about 50,000 g/mol and is available from ASI. BF-13, which is a borate (boron) free guar of charge density of about 1.1 meq/g and molecular weight of about 800,000 and BF-17, which is a borate (boron) free guar of charge density of about 1.7 5 meq/g and M. Wt. of about 800,000 both available from ASI.

The hair care compositions of the present invention may comprise a galactomannan polymer derivative having a mannose to galactose ratio of greater than 2:1 on a monomer to monomer basis, the galactomannan polymer derivative selected from the group consisting of a cationic galactomannan polymer derivative and an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers are present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of $\beta$ (1-4) glycosidic linkages. The galactose branching arises by way of an $\alpha$ (1-6) linkage. The ratio of mannose monomers to galactose monomers varies according to the species of the plant and also is affected by climate. Non Guar Galactomannan polymer derivatives of the present invention have a ratio of mannose to galactose of greater than 2:1 on a monomer to monomer basis. Suitable ratios of mannose to galactose can be greater than about 3:1, and the ratio of mannose to galactose can be greater than about 4:1. Analysis of mannose to galactose ratios is well known in the art and is typically based on the measurement of the galactose content.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose/1 part galactose), Locust bean or Carob (4 parts mannose/1 part galactose), and Cassia gum (5 parts mannose/1 part galactose).

The non-guar galactomannan polymer derivatives may have a M. Wt. from about 1,000 to about 10,000,000, and/or from about 5,000 to about 3,000,000.

The hair care compositions of the invention can also include galactomannan polymer derivatives which have a cationic charge density from about 0.5 meq/g to about 7 meq/g. The galactomannan polymer derivatives may have a cationic charge density from about 1 meq/g to about 5 meq/g. The degree of substitution of the cationic groups onto the galactomannan structure should be sufficient to provide the requisite cationic charge density.

The galactomannan polymer derivative can be a cationic derivative of the non-guar galactomannan polymer, which is obtained by reaction between the hydroxyl groups of the polygalactomannan polymer and reactive quaternary ammonium compounds. Suitable quaternary ammonium compounds for use in forming the cationic galactomannan polymer derivatives include those conforming to the general formulas 1-5, as defined above.

Cationic non-guar galactomannan polymer derivatives formed from the reagents described above are represented by the general formula 6:

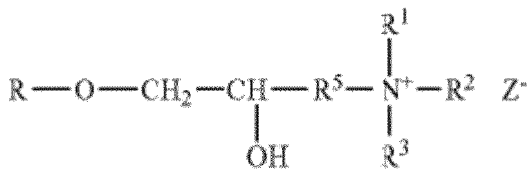

wherein R is the gum. The cationic galactomannan derivative can be a gum hydroxypropyltrimethylammonium chloride, which can be more specifically represented by the general formula 7:

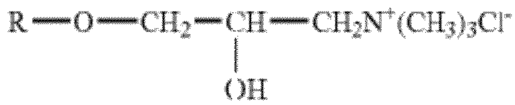

Alternatively the galactomannan polymer derivative can be an amphoteric galactomannan polymer derivative having a net positive charge, obtained when the cationic galactomannan polymer derivative further comprises an anionic group.

The cationic non-guar galactomannan can have a ratio of mannose to galactose is greater than about 4:1, a molecular weight of about 1,000 g/mol to about 10,000,000 g/mol, and/or from about 50,000 g/mol to about 1,000,000 g/mol, and/or from about 100,000 g/mol to about 900,000 g/mol, and/or from about 150,000 g/mol to about 400,000 g/mol and a cationic charge density from about 1 meq/g to about 5 meq/g, and/or from 2 meq/ g to about 4 meq/ g and can be derived from a cassia plant.

The hair care compositions can comprise water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The cationically modified starch polymers disclosed herein have a percent of bound nitrogen of from about 0.5% to about 4%.

The cationically modified starch polymers for use in the hair care compositions can have a molecular weight

about 850,000 g/mol to about 1,500,000 g/mol and/or from about 900,000 g/mol to about 1,500,000 g/mol.

The hair care compositions can include cationically modified starch polymers which have a charge density of from about 0.2 meq/g to about 5 meq/g, and/or from about 0.2 meq/g to about 2 meq/g. The chemical modification to obtain such a charge density includes, but is not limited to, the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O. B., Ed., CRC Press, Inc., Boca Raton, Fla. 1986, pp 113-125. The cationic groups may be added to the starch prior to degradation to a smaller molecular weight or the cationic groups may be added after such modification.

The cationically modified starch polymers generally have a degree of substitution of a cationic group from about 0.2 to about 2.5. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (".sup.1H NMR") methods well known in the art. Suitable .sup.1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The source of starch before chemical modification can be chosen from a variety of sources such as tubers, legumes, cereal, and grains. Non-limiting examples of this source starch may include corn starch, wheat starch, rice starch, waxy corn starch, oat starch, cassava starch, waxy barley, waxy rice starch, glutenous rice starch, sweet rice starch, amioca, potato starch, tapioca starch, oat starch, sago starch, sweet rice, or mixtures thereof.

The cationically modified starch polymers can be selected from degraded cationic maize starch, cationic tapioca, cationic potato starch, and mixtures thereof. Alternatively, the cationically modified starch polymers are cationic corn starch and cationic tapioca.

The starch, prior to degradation or after modification to a smaller molecular weight, may comprise one or more additional modifications. For example, these modifications may include cross-linking, stabilization reactions, phosphorylations, and hydrolyzations. Stabilization reactions may include alkylation and esterification.

The cationically modified starch polymers may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

An optimal form of the starch is one which is readily soluble in water and forms a substantially clear (% Transmittance of about 80 at 600 nm) solution in water. The transparency of the composition is measured by Ultra-Violet/Visible (UV/VIS) spectrophotometry, which determines the absorption or transmission of UV/VIS light by a sample, using a Gretag Macbeth Colorimeter Color i 5 according to the related instructions. A light wavelength of 600 nm has been shown to be adequate for characterizing the degree of clarity of cosmetic compositions.

Suitable cationically modified starch for use in hair care compositions are available from known starch suppliers. Also suitable for use in hair care compositions are nonionic modified starch that can be further derivatized to a cationically modified starch as is known in the art. Other suitable modified starch starting materials may be quaternized, as is known in the art, to produce the cationically modified starch polymer suitable for use in hair care compositions.

Starch Degradation Procedure: a starch slurry can be prepared by mixing granular starch in water. The temperature is raised to about 35°C. An aqueous solution of potassium permanganate is then added at a concentration of about 50 ppm based on starch. The pH is raised to about 11.5 with sodium hydroxide and the slurry is stirred sufficiently to prevent settling of the starch. Then, about a 30% solution of hydrogen peroxide diluted in water is added to a level of about 1% of peroxide based on starch. The pH of about 11.5 is then restored by adding additional sodium hydroxide. The reaction is completed over about a 1 to about 20 hour period. The mixture is then neutralized with dilute hydrochloric acid. The degraded starch is recovered by filtration followed by washing and drying.

The hair care composition can comprise a cationic copolymer of an acrylamide monomer and a cationic monomer, wherein the copolymer has a charge density of from about 1.0 meq/g to about 3.0 meq/g. The cationic copolymer can be a synthetic cationic copolymer of acrylamide monomers and cationic monomers.

The cationic copolymer can comprise:

(i) an acrylamide monomer of the following Formula AM:

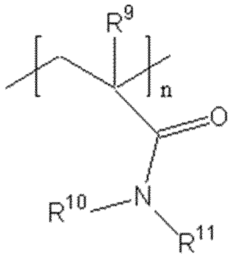

Formula AM

where $R^9$ is H or $C_{1-4}$ alkyl; and $R^{10}$ and $R^{11}$ are independently selected from the group consisting of H, $C_{1-4}$ alkyl, $CH_2OCH_3$, $CH_2OCH_2CH(CH_3)_2$, and phenyl, or together are $C_{3-6}$cycloalkyl; and
(ii) a cationic monomer conforming to Formula CM:

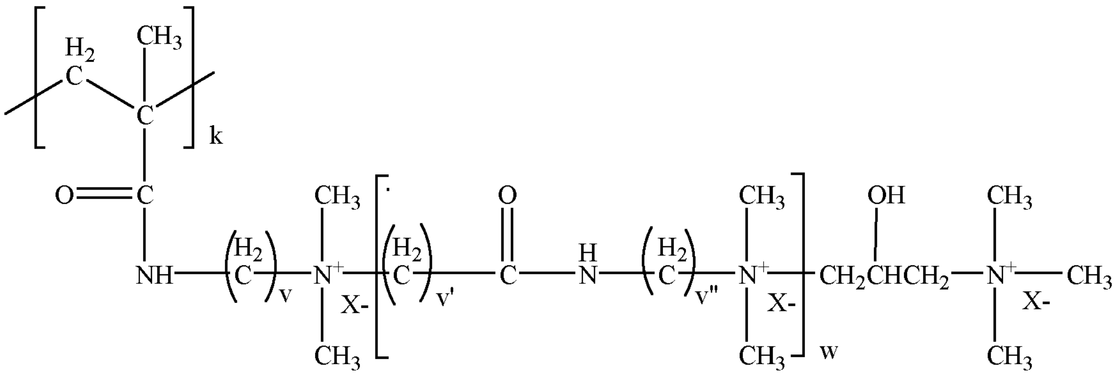

Formula CM

where k = 1, each of v, v', and v" is independently an integer of from 1 to 6, w is zero or an integer of from 1 to 10, and $X^-$ is an anion.

The cationic monomer can conform to Formula CM and where k = 1, v = 3 and w = 0, z = 1 and $X^-$ is $Cl^-$ to form the following structure:

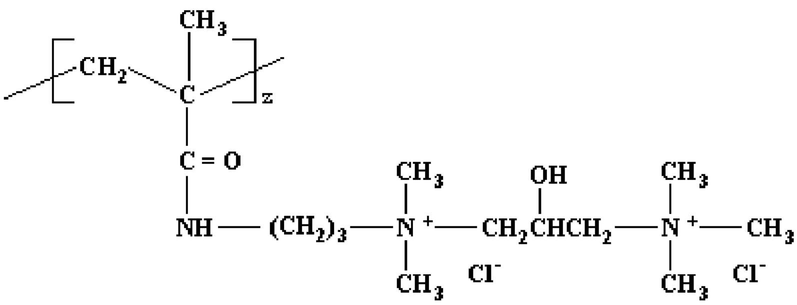

The above structure may be referred to as diquat. Alternatively, the cationic monomer can conform to Formula CM and wherein v and v" are each 3, v' = 1, w =1, y = 1 and $X^-$ is $Cl^-$, such as:

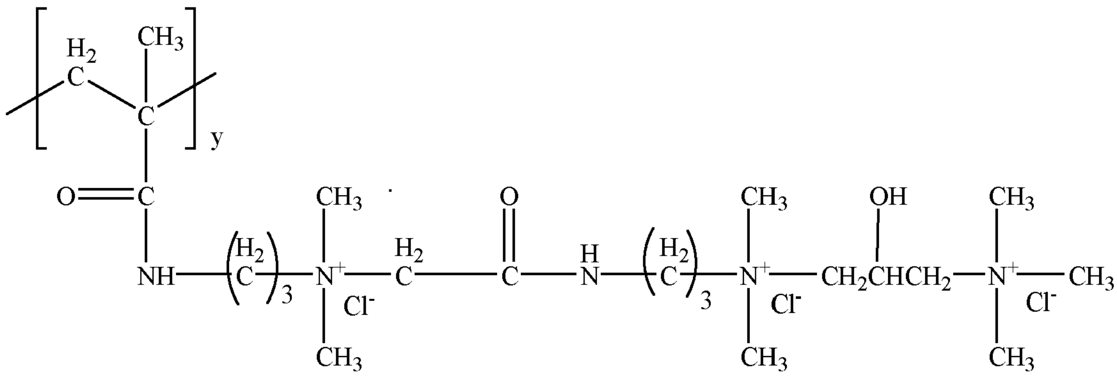

The above structure may be referred to as triquat.

Suitable acrylamide monomer include, but are not limited to, either acrylamide or methacrylamide.

The cationic copolymer (b) can be AM:TRIQUAT which is a copolymer of acrylamide and 1,3-Propanediaminium,N-[2-[[[dimethyl[3-[(2-methyl-1-oxo-2-propenyl)amino]propyl]ammonio]acetyl]amino]ethyl]2-hydroxy-*N,N,N',N',N'*-pentamethyl-, trichloride. AM:TRIQUAT is also known as polyquaternium 76 (PQ76). AM:TRIQUAT may have a charge density of 1.6 meq/g and a molecular weight of 1.1 million g/mol.

The cationic copolymer may be an acrylamide monomer and a cationic monomer, wherein the cationic monomer is selected from the group consisting of: dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide; ethylenimine, vinylamine, 2-vinylpyridine, 4- vinylpyridine; trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride, and mixtures thereof.

The cationic copolymer can comprise a cationic monomer selected from the group consisting of: cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, and mixtures thereof.

The cationic copolymer can be water-soluble. The cationic copolymer is formed from (1) copolymers of (meth) acrylamide and cationic monomers based on (meth)acrylamide, and/or hydrolysis-stable cationic monomers, (2) terpolymers of (meth)acrylamide, monomers based on cationic (meth)acrylic acid esters, and monomers based on (meth)acrylamide, and/or hydrolysis-stable cationic monomers. Monomers based on cationic (meth)acrylic acid esters may be cationized esters of the (meth)acrylic acid containing a quaternized N atom. The cationized esters of the (meth) acrylic acid containing a quaternized N atom may be quaternized dialkylaminoalkyl (meth)acrylates with C1 to C3 in the alkyl and alkylene groups. Suitable cationized esters of the (meth)acrylic acid containing a quaternized N atom can be selected from the group consisting of: ammonium salts of dimethylaminomethyl (meth)acrylate, dimethylaminoethyl (meth)acrylate, dimethylaminopropyl (meth)acrylate, diethylaminomethyl (meth)acrylate, diethylaminoethyl (meth)acrylate; and diethylaminopropyl (meth)acrylate quaternized with methyl chloride. The cationized esters of the (meth)acrylic acid containing a quaternized N atom may be dimethylaminoethyl acrylate, which is quaternized with an alkyl halide, or with methyl chloride or benzyl chloride or dimethyl sulfate (ADAME-Quat). the cationic monomer when based on (meth) acrylamides can be quaternized dialkylaminoalkyl(meth)acrylamides with C1 to C3 in the alkyl and alkylene groups, or dimethylaminopropylacrylamide, which is quaternized with an alkyl halide, or methyl chloride or benzyl chloride or dimethyl sulfate.

Suitable cationic monomer based on a (meth)acrylamide include quaternized dialkylaminoalkyl(meth)acrylamide with C1 to C3 in the alkyl and alkylene groups. The cationic monomer based on a (meth)acrylamide can be dimethylaminopropylacrylamide, which is quaternized with an alkyl halide, especially methyl chloride or benzyl chloride or dimethyl sulfate.

The cationic monomer can be a hydrolysis-stable cationic monomer. Hydrolysis-stable cationic monomers can be, in addition to a dialkylaminoalkyl(meth)acrylamide, all monomers that can be regarded as stable to the OECD hydrolysis test. The cationic monomer can be hydrolysis-stable and the hydrolysis-stable cationic monomer can be selected from the group consisting of: diallyldimethylammonium chloride and water-soluble, cationic styrene derivatives.

The cationic copolymer can be a terpolymer of acrylamide, 2-dimethylammoniumethyl (meth)acrylate quaternized with methyl chloride (ADAME-Q) and 3-dimethylammoniumpropyl(meth)acrylamide quaternized with methyl chloride (DIMAPA-Q). The cationic copolymer can be formed from acrylamide and acrylamidopropyltrimethylammonium

chloride, wherein the acrylamidopropyltrimethylammonium chloride has a charge density of from about 1.0 meq/g to about 3.0 meq/g.

The cationic copolymer can have a charge density of from about 1.1 meq/g to about 2.5 meq/g, or from about 1.1 meq/g to about 2.3 meq/g, or from about 1.2 meq/g to about 2.2 meq/g, or from about 1.2 meq/g to about 2.1 meq/g, or from about 1.3 meq/g to about 2.0 meq/g, or from about 1.3 meq/g to about 1.9 meq/g.

The cationic copolymer can have a molecular weight from about 100 thousand g/mol to about 1.5 million g/mol, or from about 300 thousand g/mol to about 1.5 million g/mol, or from about 500 thousand g/mol to about 1.5 million g/mol, or from about 700 thousand g/mol to about 1.0 million g/mol, or from about 900 thousand g/mol to about 1.2 million g/mol.

The cationic copolymer can be a trimethylammoniopropylmethacrylamide chloride-N-Acrylamide copolymer, which is also known as AM:MAPTAC. AM:MAPTAC may have a charge density of about 1.3 meq/g and a molecular weight of about 1.1 million g/mol. The cationic copolymer can be AM:ATPAC. AM:ATPAC can have a charge density of about 1.8 meq/g and a molecular weight of about 1.1 million g/mol.

(a) Cationic Synthetic Polymers

The hair care composition can comprise a cationic synthetic polymer that may be formed from

i) one or more cationic monomer units, and optionally
ii) one or more monomer units bearing a negative charge, and/or
iii) a nonionic monomer,

wherein the subsequent charge of the copolymer is positive. The ratio of the three types of monomers is given by "m", "p" and "q" where "m" is the number of cationic monomers, "p" is the number of monomers bearing a negative charge and "q" is the number of nonionic monomers

The cationic polymers can be water soluble or dispersible, non-crosslinked, and synthetic cationic polymers having the following structure:

Monomer bearing a negative charge

Cationic moiety

Nonionic monomer

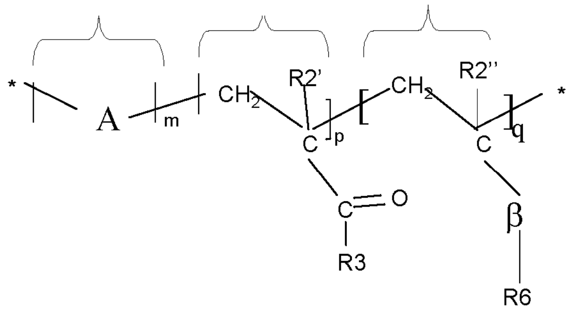

m ≥ 1
p=0 or ≥ 1
q=0 or ≥ 1
m ≥ p

where A, may be one or more of the following cationic moieties:

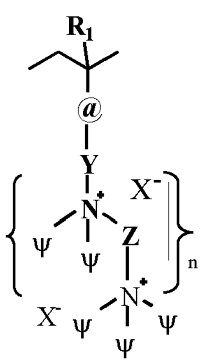

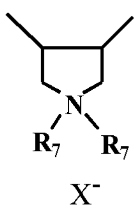

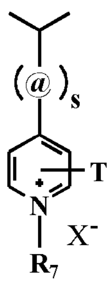

where @ = amido, alkylamido, ester, ether, alkyl or alkylaryl;
where Y = C1-C22 alkyl, alkoxy, alkylidene, alkyl or aryloxy;

where ψ = C1-C22 alkyl, alkyloxy, alkyl aryl or alkyl arylox;.
where Z = C1-C22 alkyl, alkyloxy, aryl or aryloxy;
where R1 = H, C1-C4 linear or branched alkyl;
where s = 0 or 1, n = 0 or ≥ 1;
where T and R7 = C1-C22 alkyl; and
where X- = halogen, hydroxide, alkoxide, sulfate or alkylsulfate.

Where the monomer bearing a negative charge is defined by R2' = H, C1-C4 linear or branched alkyl and R3 as:

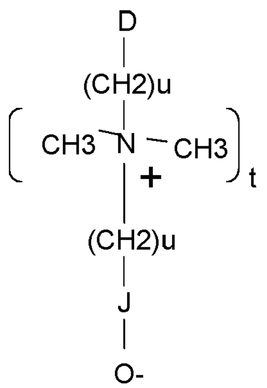 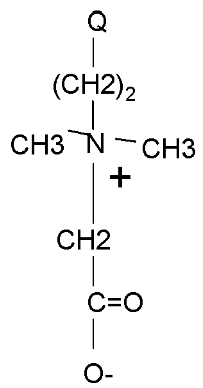 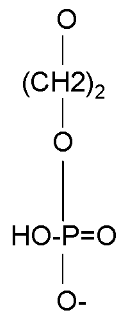 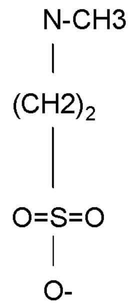

where D = O, N, or S;
where Q = $NH_2$ or O;
where u = 1-6;
where t = 0-1; and
where J = oxygenated functional group containing the following elements P, S, C.

Where the nonionic monomer is defined by R2'' = H, C1-C4 linear or branched alkyl, R6 = linear or branched alkyl, alkyl aryl, aryl oxy, alkyloxy, alkylaryl oxy and β is defined as

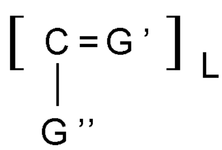

;

and where G' and G'' are, independently of one another, O, S or N-H and L =0 or 1.

Examples of cationic monomers include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving from therefrom.

Further examples of cationic monomers include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.

Suitable cationic monomers include those which comprise a quaternary ammonium group of formula $-NR_3^+$, wherein R, which is identical or different, represents a hydrogen atom, an alkyl group comprising 1 to 10 carbon atoms, or a benzyl group, optionally carrying a hydroxyl group, and comprise an anion (counter-ion). Examples of anions are halides such as chlorides, bromides, sulphates, hydrosulphates, alkylsulphates (for example comprising 1 to 6 carbon atoms), phosphates, citrates, formates, and acetates.

Suitable cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl

(meth)acrylamido chloride, vinylbenzyl trimethyl ammonium chloride.

Additional suitable cationic monomers include trimethyl ammonium propyl (meth)acrylamido chloride.

Examples of monomers bearing a negative charge include alpha ethylenically unsaturated monomers comprising a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.

Suitable monomers with a negative charge include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Suitable nonionic monomers include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion (X- ) in association with the synthetic cationic polymers may be any known counterion so long as the polymers remain soluble or dispersible in water, in the hair care composition, or in a coacervate phase of the hair care composition, and so long as the counterions are physically and chemically compatible with the essential components of the hair care composition or do not otherwise unduly impair product performance, stability or aesthetics. Non limiting examples of such counterions include halides (e.g., chlorine, fluorine, bromine, iodine), sulfate and methylsulfate.

The cationic polymer described herein can aid in providing damaged hair, particularly chemically treated hair, with a surrogate hydrophobic F-layer. The microscopically thin F-layer provides natural weatherproofing, while helping to seal in moisture and prevent further damage. Chemical treatments damage the hair cuticle and strip away its protective F-layer. As the F-layer is stripped away, the hair becomes increasingly hydrophilic. It has been found that when lyotropic liquid crystals are applied to chemically treated hair, the hair becomes more hydrophobic and more virgin-like, in both look and feel. Without being limited to any theory, it is believed that the lyotropic liquid crystal complex creates a hydrophobic layer or film, which coats the hair fibers and protects the hair, much like the natural F-layer protects the hair. The hydrophobic layer returns the hair to a generally virgin-like, healthier state. Lyotropic liquid crystals are formed by combining the synthetic cationic polymers described herein with the aforementioned anionic detersive surfactant component of the hair care composition. The synthetic cationic polymer has a relatively high charge density. It should be noted that some synthetic polymers having a relatively high cationic charge density do not form lyotropic liquid crystals, primarily due to their abnormal linear charge densities. Such synthetic cationic polymers are described in WO 94/06403 to Reich et al. The synthetic polymers described herein can be formulated in a stable hair care composition that provides improved conditioning performance, with respect to damaged hair.

Cationic synthetic polymers that can form lyotropic liquid crystals have a cationic charge density of from about 2 meq/gm to about 7 meq/gm, and/or from about 3 meq/gm to about 7 meq/gm, and/or from about 4 meq/gm to about 7 meq/gm. The cationic charge density may be about 6.2 meq/gm. The polymers may also have a M. Wt. of from about 1,000 to about 5,000,000, and/or from about 10,000 to about 1,500,000, and/or from about 100,000 to about 1,500,000.

The cationic synthetic polymers that provide enhanced conditioning and deposition of benefit agents but do not necessarily form lyotropic liquid crystals may have a cationic charge density of from about 0.7 meq/gm to about 7 meq/gm, and/or from about 0.8 meq/gm to about 5 meq/gm, and/or from about 1.0 meq/gm to about 3 meq/gm. The polymers also have a M. Wt. of from about 1,000 to about 1,500,000, from about 10,000 to about 1,500,000, and from about 100,000 to about 1,500,000.

Suitable cationic cellulose polymers are salts of hydroxyethyl cellulose reacted with trimethyl ammonium substituted epoxide, referred to in the industry (CTFA) as Polyquaternium 10 and available from Dow/ Amerchol Corp. (Edison, N.J., USA) in their Polymer LR, JR, and KG series of polymers. Non-limiting examples include: JR-30M, KG-30M, JP, LR-400 and mixtures thereof. Other suitable types of cationic cellulose include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available from Dow/ Amerchol Corp. under the tradename SoftCAT Polymer SL-5, SoftCAT Polymer SL-30, Polymer SL-60, Polymer SL-100, Polymer SK-L, Polymer

SK-M, Polymer SK-MH, and Polymer SK-H.

The concentration of the cationic polymers ranges about 0.01% to about 5%, from about 0.08% to about 3%, from about 0.1% to about 2%, and/or from about 0.2% to about 1%, by weight of the hair care composition.

ANIONIC POLYMERS

The hair care composition may also comprises one or more anionic polymers. Anionic polymers are polymers that contain anionic functional groups. Non-limited examples of anionic functional groups include carboxylate, sulfonate, sulfate, phosphonate, phosphate, nitrate and others. Anionic polymers can be homopolymers, that is, polymers constructed using one type of monomer, or copolymers, that is, polymers constructed using more than one type of monomer (including one or more monomers with anionic functional groups and/or other monomers that contain non anionic functional groups). The hair care composition comprises from about 1% to about 2.5% of an anionic polymer.

The anionic polymer may be a polyacrylate, or polyacrylamide polymer. The hair care composition may comprise anionic polymers that are homopolymers based on acrylic acid, methacrylic acid or other related derivatives, non-limiting examples include polyacrylate, polymethacrylate, polyethylacrylate, and polyacrylamide.

The anionic polymer may be alkali swellable and hydrophobically-modified alkali swellable acrylic copolymers or methacrylate copolymers, non-limiting examples include acrylic acid/acrylonitrogens copolymer, acrylates/steareth-20 itaconate copolymer, acrylates/ceteth-20 itaconate copolymer, Acrylates/Aminoacrylates/C10-30 Alkyl PEG-20 Itaconate Copolymer, acrylates/aminoacrylates copolymer, acrylates/steareth-20 methacrylate copolymer, acrylates/beheneth-25 methacrylate copolymer, acrylates/steareth-20 methacrylate crosspolymer, acrylates/beheneth-25 methacrylate/HEMA crosspolymer, acrylates/vinyl neodecanoate crosspolymer, acrylates/vinyl isodecanoate crosspolymer, Acrylates/Palmeth-25 Acrylate Copolymer, Acrylic Acid/ Acrylamidomethyl Propane Sulfonic Acid Copolymer, and acrylates/C10-C30 alkyl acrylate crosspolymer.

The anionic polymer may be soluble crosslinked acrylic polymers, a non-limiting example includes carbomers.

The anionic polymer may be an associative polymer, non-limiting examples include: hydrophobically modified, alkali swellable emulsions, non-limiting examples include hydrophobically modified polypolyacrylates; hydrophobically modified polyacrylic acids, and hydrophobically modified polyacrylamides; hydrophobically modified polyethers wherein these materials may have a hydrophobe that can be selected from cetyl, stearyl, oleayl, and combinations thereof.

Non-limited examples of anionic polymers include: C-LC/SD-PC: Sodium Polyaspartate; Poly(2-acrylamido-2-methyl-1-propanesulfonic acid) which has a MW of about 2,000,000; Poly(2-acrylamido-2-methyl-1-propanesulfonic acid-co-acrylonitrile) acrylonitrile; Poly(4-styrenesulfonic acid-co-maleic acid) sodium salt which has a MW of about 20,000; PVM/MA Copolymer; FLEXAN® II Polymer: Sodium Polystyrene Sulfonate which has a MW of about 70,000 or 200,000 or 1,000,000; Polystyrene Sulfonate which has a MW of about 75,000; Ammonium Polystyrene Sulfonate which has a MW of about 200,000; MIRUSTYLE X-HP Sodium Laneth-40 Maleate / Styrene Sulfonate Copolymer; MIRUSTYLE X-HV Sodium Methoxy PEG-16 Maleate / Styrene Sulfonate Copolymer; Polyanetholesulfonic acid sodium salt; Covacryl MV 60: Sodium Polyacrylate which has a MW of about 2,100 or 5,100 or 8,000 or 15,000; Covacryl SP: Polyacrylic Acid; Poly(vinyl sulfate) potassium salt which has a MW of about 170,000; Poly(vinylsulfonic acid, sodium salt); EcoSmooth™ Satin: Ethylene/Sodium Acrylate Copolymer; Fixate™ Plus Polymer: Polyacrylate-14; Fixate™ Superhold Polymer: Polyacrylate-2 Crosspolymer; Fixomer™ A-30 Polymer: Methacrylic Acid (and) Methacrylic Acid/Sodium Acrylamidomethyl Propane Sulfonate Copolymer; Hyafactor™-PGA: Sodium Polyglutamate; Itaconix® DSP 2K™: Sodium Polyitaconate; Sodium Acrylate/Sodium Acryloyldimethyl Taurate Copolymer; Luviset® CA 66: VA/Crotonates Copolymer; Luviset® CAN: VA/Crotonates/Vinyl Neodecanoate Copolymer; Acrylates/Methacrylamide Copolymer; Carbomer; Sodium Carbomer; Rheocare® HSP-1180: Polyacrylamidomethylpropane Sulfonic Acid; Sodium Polynaphthalenesulfonate; Blanose™ cellulose gum purified sodium carboxymethylcellulose (CMC): Cellulose Gum; Aqualon® cellulose gum (CMC): Cellulose Gum; Fixate™ Design Polymer: Polyacrylate-32; Rhodicare® CFT: Xanthan Gum; Luviset® CAN: VA/Crotonates/Vinyl Neodecanoate Copolymer; Fixomer™ A-30 Polymer: Methacrylic Acid (and) Methacrylic Acid/Sodium Acrylamidomethyl Propane Sulfonate Copolymer; Fucogel®: Biosaccharide Gum-1; PECOSIL® PS-112: Dimethicone PEG-7 Phosphate; Ecopol-18S Anionic Guar: Carboxymethyl Hydroxypropyl Guar; Carrageenan; Sodium Carrageenan; Poly(methacrylic acid) with MW of about 100,000; Poly(ethyl acrylate/acrylic acid); Poly(methyl methacrylate/-methacrylic acid) [90:10 monomer ratio] with MW of about 100,000; Poly(methyl methacrylate/methacrylic acid) [75:25 monomer ratio] with MW of about 1.2 million; Poly(methyl methacrylate/methacrylic acid) with MW of about 1.2 million 500,000; Poly(methyl methacrylate/methacrylic acid) [80:20 monomer ratio]; Poly(styrenesulfonic acid/maleic acid), sodium salt with MW of about 15,000; Poly(methacrylic acid) ammonium salt, 30% solution in water with MW of about 15,000; Poly(butadiene/maleic acid) 1:1, 42% soln. in water with MW of about 10,000-15,000; Poly(maleic acid), 50% soln. in water with MW of about 800-1200; Poly(vinylphosphonic acid), 30% Solution with MW of about 24,000; Poly(vinyl phosphoric acid), sodium salt with MW of about 200,000; Poly(vinylsulfonic acid) sodium salt, 25% solution in water with MW of about 4,000-6,000; Poly(acrylic acid), powder with MW of about 4,000,000; Poly(acrylic acid), 50% solution water with MW of about 5,000; Poly(acrylic acid), sodium salt, powder with MW of about 2,000; Poly(acrylic acid), sodium salt,

40% solution with MW of about 3,000; all available from Polysciences, Inc.

**Gel Network**

In the present invention, a gel network may be present. The gel network component of the present invention may comprise at least one fatty amphiphile. As used herein, "fatty amphiphile" refers to a compound having a hydrophobic tail group as defined as an alkyl, alkenyl (containing up to 3 double bonds), alkyl aromatic, or branched alkyl group of $C_{12}$-$C_{70}$ length and a hydrophilic head group which does not make the compound water soluble, wherein the compound also has a net neutral charge at the pH of the shampoo composition.

The shampoo compositions of the present invention comprise fatty amphiphile as part of the pre-formed dispersed gel network phase in an amount from about 0.05% to about 14%, preferably from about 0.5% to about 10%, and more preferably from about 1% to about 8%, by weight of the shampoo composition.

According to the present invention, suitable fatty amphiphiles, or suitable mixtures of two or more fatty amphiphiles, have a melting point of at least about 27°C. The melting point, as used herein, may be measured by a standard melting point method as described in U.S. Pharmacopeia, USP-NF General Chapter <741> "Melting range or temperature". The melting point of a mixture of two or more materials is determined by mixing the two or more materials at a temperature above the respective melt points and then allowing the mixture to cool. If the resulting composite is a homogeneous solid below about 27°C, then the mixture has a suitable melting point for use in the present invention. A mixture of two or more fatty amphiphiles, wherein the mixture comprises at least one fatty amphiphile having an individual melting point of less than about 27°C, still is suitable for use in the present invention provided that the composite melting point of the mixture is at least about 27°C.

Suitable fatty amphiphiles of the present invention include fatty alcohols, alkoxylated fatty alcohols, fatty phenols, alkoxylated fatty phenols, fatty amides, alkyoxylated fatty amides, fatty amines, fatty alkylamidoalkylamines, fatty alkyoxyalted amines, fatty carbamates, fatty amine oxides, fatty acids, alkoxylated fatty acids, fatty diesters, fatty sorbitan esters, fatty sugar esters, methyl glucoside esters, fatty glycol esters, mono, di & tri glycerides, polyglycerine fatty esters, alkyl glyceryl ethers, propylene glycol fatty acid esters, cholesterol, ceramides, fatty silicone waxes, fatty glucose amides, and phospholipids and mixtures thereof.

In the present invention, the shampoo composition may comprise fatty alcohol gel networks. These gel networks are formed by combining fatty alcohols and surfactants in the ratio of from about 1:1 to about 40:1, from about 2:1 to about 20:1, and/or from about 3:1 to about 10:1. The formation of a gel network involves heating a dispersion of the fatty alcohol in water with the surfactant to a temperature above the melting point of the fatty alcohol. During the mixing process, the fatty alcohol melts, allowing the surfactant to partition into the fatty alcohol droplets. The surfactant brings water along with it into the fatty alcohol. This changes the isotropic fatty alcohol drops into liquid crystalline phase drops. When the mixture is cooled below the chain melt temperature, the liquid crystal phase is converted into a solid crystalline gel network. The gel network contributes a stabilizing benefit to cosmetic creams and hair conditioners. In addition, they deliver conditioned feel benefits for hair conditioners.

The fatty alcohol can be included in the fatty alcohol gel network at a level by weight of from about 0.05 wt% to about 14 wt%. For example, the fatty alcohol may be present in an amount ranging from about 1 wt% to about 10 wt%, and/or from about 6 wt% to about 8 wt%.

The fatty alcohols useful herein include those having from about 10 to about 40 carbon atoms, from about 12 to about 22 carbon atoms, from about 16 to about 22 carbon atoms, and/or about 16 to about 18 carbon atoms. These fatty alcohols can be straight or branched chain alcohols and can be saturated or unsaturated. Nonlimiting examples of fatty alcohols include cetyl alcohol, stearyl alcohol, behenyl alcohol, and mixtures thereof. Mixtures of cetyl and stearyl alcohol in a ratio of from about 20:80 to about 80:20 are suitable.

Gel network preparation: A vessel is charged with water and the water is heated to about 74°C. Cetyl alcohol, stearyl alcohol, and SLES surfactant are added to the heated water. After incorporation, the resulting mixture is passed through a heat exchanger where the mixture is cooled to about 35°C. Upon cooling, the fatty alcohols and surfactant crystallized to form a crystalline gel network. Table 1 provides the components and their respective amounts for an example gel network composition.

Table 1

Gel network components

| Ingredient | Wt. % |
|---|---|
| Water | 78.27% |
| Cetyl Alcohol | 4.18% |
| Stearyl Alcohol | 7.52% |

(continued)

Gel network components

| Ingredient | Wt. % |
|---|---|
| Sodium laureth-3 sulfate (28% Active) | 10.00% |
| 5-Chloro-2-methyl-4-isothiazolin-3-one, Kathon CG | 0.03% |

1. WATER MISCIBLE SOLVENTS

The carrier useful in the hair care composition may include water and water solutions of lower alkyl alcohols, polyhydric alcohols, ketones having from 3 to 4 carbons atoms, C1-C6 esters of C1-C6 alcohols, sulfoxides, amides, carbonate esters, ethoxylated and proposylated C1-C10 alcohols, lactones, pyrollidones, and mixtures thereof. Non-limited lower alkyl alcohol examples are monohydric alcohols having 1 to 6 carbons, such as ethanol and isopropanol. Non-limiting examples of polyhydric alcohols useful herein include propylene glycol, dipropylene glycol, butylenes glycol, hexylene glycol, glycerin, propane diol and mixtures thereof.

The hair care composition may comprise a hydrotrope/viscosity modifier which is an alkali metal or ammonium salt of a lower alkyl benzene sulphonate such as sodium xylene sulphonate, sodium cumene sulphonate or sodium toluene sulphonate.

In the present invention, the hair care composition may comprise silicone/PEG-8 silicone/PEG-9 silicone/PEG-n silicone/silicone ether (n could be another integer), non-limiting examples include PEG8-dimethicone A208) MW 855, PEG 8 Dimethicone D208 MW 2706.

C. PROPELLANT OR BLOWING AGENT

The hair care composition described herein may comprise from about from about 1% to about 10% propellant or blowing agent, alternatively from about 2% to about 8% propellant, by weight of the hair care composition.

The propellant or blowing agent may comprise one or more volatile materials, which in a gaseous state, may carry the other components of the hair care composition in particulate or droplet form or as a foam. The propellant or blowing agent may have a boiling point within the range of from about -45° C. to about 5° C. The propellant or blowing agent may be liquefied when packaged in convention aerosol containers under pressure. The rapid boiling of the propellant or blowing agent upon leaving the aerosol foam dispenser may aid in the atomization or foaming of the other components of the hair care composition.

Aerosol propellants or blowing agents which may be employed in the aerosol composition may include the chemically-inert hydrocarbons such as propane, n-butane, isobutane, cyclopropane, and mixtures thereof, as well as halogenated hydrocarbons such as dichlorodifluoromethane, 1,1-dichloro-1,1,2,2-tetrafluoroethane, 1-chloro-1,1-difluoro-2,2-trifluoroethane, 1-chloro-1,1-difluoroethylene, 1,1-difluoroethane, dimethyl ether, monochlorodifluoromethane, trans-1,3,3,3-tetrafluoropropene, and mixtures thereof. The propellant or blowing agent may comprise hydrocarbons such as isobutane, propane, and butane-these materials may be used for their low ozone reactivity and may be used as individual components where their vapor pressures at 21.1° C. range from about 1.17 Bar to about 7.45 Bar, alternatively from about 1.17 Bar to about 4.83 Bar, and alternatively from about 2.14 Bar to about 3.79 Bar.

D. SCALP HEALTH AGENTS

In the present invention, one or more scalp health agent may be added to provide scalp benefits in addition to the anti-fungal/anti-dandruff efficacy provided by the surfactant soluble anti-dandruff agents. This group of materials is varied and provides a wide range of benefits including moisturization, barrier improvement, anti-fungal, anti-microbial and anti-oxidant, anti-itch, and sensates, and additional anti-dandruff agents such as polyvalent metal salts of pyrithione, non-limiting examples include zinc pyrithione (ZPT) and copper pyrithione, sulfur, or selenium sulfide. Such scalp health agents include but are not limited to: vitamin E and F, niacinamide, caffeine, panthenol, zinc oxide, zinc carbonate, basic zinc carbonate, glycols, glycolic acid, PCA, PEGs, erythritol, glycerin, triclosan, lactates, hyaluronates, allantoin and other ureas, betaines, sorbitol, glutamates, xylitols, menthol, menthyl lactate, iso cyclomone, benzyl alcohol, a compound comprising the following structure:

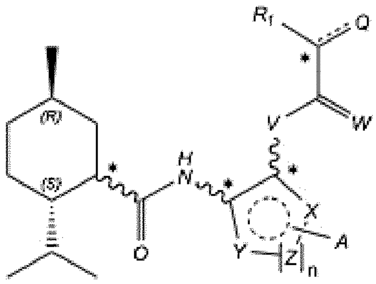

$R_1$ is selected from H, alkyl, amino alkyl, alkoxy;
Q = $H_2$, O, $-OR_1$, $-N(R_1)_2$, $-OPO(OR_1)_x$, $-PO(OR_1)_x$, $-P(OR_1)_x$ where x = 1-2;
V = $NR_1$, O, $-OPO(OR_l)_X$, $-PO(OR_1)_x$, $-P(OR_1)_x$ where x = 1-2;
W = $H_2$, O;
X, Y = independently selected from H, aryl, naphthyl for n=0;
X, Y = aliphatic $CH_2$ or aromatic CH for n ≥ 1 and Z is selected from aliphatic $CH_2$, aromatic CH, or heteroatom;
A = lower alkoxy, lower alkylthio, aryl, subsitituted aryl or fused aryl; and
stereochemistry is variable at the positions marked*.
and natural extracts/oils including peppermint, spearmint, argan, jojoba and aloe.

E. OPTIONAL INGREDIENTS

In the present invention, the hair care composition may further comprise one or more optional ingredients, including benefit agents Suitable benefit agents include, but are not limited to conditioning agents, cationic polymers silicone emulsions, anti-dandruff agents, gel networks, chelating agents, and, natural oils such as sun flower oil or castor oil. Additional suitable optional ingredients include but are not limited to perfumes, perfume microcapsules, colorants, particles, anti-microbials, foam busters, anti-static agents, rheology modifiers and thickeners, suspension materials and structurants, pH adjusting agents and buffers, preservatives, pearlescent agents, solvents, diluents, anti-oxidants, vitamins and combinations thereof. In the present invention, the composition may have from about 0.5% to about 7% of a perfume.

Such optional ingredients should be physically and chemically compatible with the components of the composition, and should not otherwise unduly impair product stability, aesthetics, or performance. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein.

1. Conditioning Agents

The conditioning agent of the hair care compositions can be a silicone conditioning agent. The silicone conditioning agent may comprise volatile silicone, non-volatile silicone, or combinations thereof. The concentration of the silicone conditioning agent typically ranges from about 0.01% to about 10%, by weight of the composition, from about 0.1% to about 8%, from about 0.1% to about 5%, and/or from about 0.2% to about 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609.

The silicone conditioning agents for use in the compositions of the present invention can have a viscosity, as measured at 25° C, from about 20 to about 2,000,000 centistokes ("csk"), from about 1,000 to about 1,800,000 csk, from about 10,000 to about 1,500,000 csk, and/or from about 20,000 to about 1,500,000 csk.

The dispersed silicone conditioning agent particles typically have a volume average particle diameter ranging from about 0.01 micrometer to about 60 micrometer. For small particle application to hair, the volume average particle diameters typically range from about 0.01 micrometer to about 4 micrometer, from about 0.01 micrometer to about 2 micrometer, from about 0.01 micrometer to about 0.5 micrometer.

Additional material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

Silicone emulsions suitable for use in the the present invention include, but are not limited to, emulsions of insoluble polysiloxanes. These may be prepared via emulsion polymerization, as in accordance with the descriptions provided in U.S. Patent No. 6,316,541 or U.S. Patent No. 4,476,282 or U.S. Patent Application Publication No. 2007/0276087, or they may be emulsified after polymerization is complete, via a variety of emulsification methods as described in U.S. Patent 9,255,184B2 or U.S. Patent 7,683,119 or Emulsions and Emulsion Stability, edited by Johan

Sjoblom, CRC Press, 2005. These references can be consulted for a non-limiting list of suitable emulsifiers and emulsifier blends based on the functionality of silicone used, the emulsification method, and the desired emulsion particle size. Accordingly, suitable insoluble polysiloxanes include polysiloxanes such as alpha, omega hydroxy-terminated polysiloxanes or alpha, omega alkoxy-terminated polysiloxanes having an internal phase viscosity from about 5 csk to about 500,000 csk. For example, the insoluble polysiloxane may have an internal phase viscosity less 400,000 csk, preferably less than 200,000 csk, more preferably from about 10,000 csk to about 180,000 csk. The insoluble polysiloxane can have an average particle size within the range from about 10 nm to about 10 micron. The average particle size may be within the range from about 15 nm to about 5 micron, from about 20 nm to about 1 micron, or from about 25 nm to about 550 nm or from about 1 to 10 micron. The concentration of dispersed silicone in the emulsion may be within the range from about 5 to 90 percent, or from 20 to 85 percent, or from 30 to 80 percent by weight of the emulsion composition.

The average molecular weight of the insoluble polysiloxane, the internal phase viscosity of the insoluble polysiloxane, the viscosity of the silicone emulsion, and the size of the particle comprising the insoluble polysiloxane are determined by methods commonly used by those skilled in the art, such as the methods disclosed in Smith, A. L. The Analytical Chemistry of Silicones, John Wiley & Sons, Inc.: New York, 1991. For example, the viscosity of the silicone emulsion can be measured at 30°C with a Brookfield viscometer with spindle 6 at 2.5 rpm. The silicone emulsion may further include an additional emulsifier together with the anionic surfactant,

Other classes of silicones suitable for use in compositions of the present invention include but are not limited to: i) silicone fluids, including but not limited to, silicone oils, which are flowable materials having viscosity less than about 1,000,000 csk as measured at 25°C; ii) aminosilicones, which contain at least one primary, secondary or tertiary amine; iii) cationic silicones, which contain at least one quaternary ammonium functional group; iv) silicone gums; which include materials having viscosity greater or equal to 1,000,000 csk as measured at 25°C; v) silicone resins, which include highly cross-linked polymeric siloxane systems; vi) high refractive index silicones, having refractive index of at least 1.46, and vii) mixtures thereof.

The conditioning agent of the hair care compositions of the present invention may also comprise at least one organic conditioning material such as oil or wax, either alone or in combination with other conditioning agents, such as the silicones described above. The organic material can be non-polymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-200, PEG-400, PEG-600, PEG-1000, PEG-2M, PEG-7M, PEG-14M, PEG-45M and mixtures thereof.

2. <u>Emusifiers</u>

A variety of anionic and nonionic emulsifiers can be used in the hair care composition of the present invention. The anionic and nonionic emulsifiers can be either monomeric or polymeric in nature. Monomeric examples include, by way of illustrating and not limitation, alkyl ethoxylates, alkyl sulfates, soaps, and fatty esters and their derivatives. Polymeric examples include, by way of illustrating and not limitation, polyacrylates, polyethylene glycols, and block copolymers and their derivatives. Naturally occurring emulsifiers such as lanolins, lecithin and lignin and their derivatives are also non-limiting examples of useful emulsifiers.

3. <u>Chelating Agents</u>

The hair care composition can also comprise a chelant. Suitable chelants include those listed in A E Martell & R M Smith, Critical Stability Constants, Vol. 1, Plenum Press, New York & London (1974) and A E Martell & R D Hancock, Metal Complexes in Aqueous Solution, Plenum Press, New York & London (1996). When related to chelants, the term "salts and derivatives thereof" means the salts and derivatives comprising the same functional structure (e.g., same chemical backbone) as the chelant they are referring to and that have similar or better chelating properties. This term include alkali metal, alkaline earth, ammonium, substituted ammonium (i.e. monoethanolammonium, diethanolammonium, triethanolammonium) salts, esters of chelants having an acidic moiety and mixtures thereof, in particular all sodium, potassium or ammonium salts. The term "derivatives" also includes "chelating surfactant" compounds, such as those exemplified in U.S. Pat. No. 5,284,972, and large molecules comprising one or more chelating groups having the same functional structure as the parent chelants, such as polymeric EDDS (ethylenediaminedisuccinic acid) disclosed in U.S. Pat. No. 5,747,440.

Chelating agents can be incorporated in the compositions herein in amounts ranging from 0.001% to 10.0% by weight of the total composition, preferably 0.01% to 2.0%.

Nonlimiting chelating agent classes include carboxylic acids, aminocarboxylic acids, including aminocids,

phosphoric acids, phosphonic acids, polyphosponic acids, polyethyleneimines, polyfunctionally-substituted aromatic, their derivatives and salts.

Nonlimiting chelating agents include the following materials and their salts. Ethylenediaminetetraacetic acid (EDTA), ethylenediaminetriacetic acid, ethylenediamine-N,N'-disuccinic acid (EDDS), ethylenediamine-N,N'-diglutaric acid (EDDG), salicylic acid, aspartic acid, glutamic acid, glycine, malonic acid, histidine, diethylenetriaminepentaacetate (DTPA), N-hydroxyethylethylenediaminetriacetate, nitrilotriacetate, ethylenediaminetetrapropionate, triethylenetetraaminehexaacetate, ethanoldiglycine, propylenediaminetetracetic acid (PDTA), methylglycinediacetic acid (MODA), diethylenetriaminepentaacetic acid, methylglycinediacetic acid (MGDA), N-acyl-N,N',N'-ethylenediaminetriacetic acid, nitrilotriacetic acid, ethylenediamineglutaric acid (EDGA), 2-hydroxypropylenediamine disuccinic acid (HPDS), glycinamide-N, N'-disuccinic acid (GADS), 2-hydroxypropylenediamine-N-N'-disuccinic acid (HPDDS), N-2-hydroxyethyl-N,N-diacetic acid, glyceryliminodiacetic acid, iminodiacetic acid-N-2-hydroxypropyl sulfonic acid, aspartic acid N-carboxymethyl-N-2-hydroxypropyl-3-sulfonic acid, alanine-N,N'-diacetic acid, aspartic acid-N,N'-diacetic acid, aspartic acid N-monoacetic acid, iminodisuccinic acid, diamine-N,N'-dipolyacid, monoamide-N,N'-dipolyacid, diaminoalkyldi(sulfosuccinic acids) (DDS), ethylenediamine-N-N'-bis (ortho-hydroxyphenyl acetic acid)), N,N'-bis(2-hydroxybenzyl)ethylenediamine-N, N'-diacetic acid, ethylenediaminetetraproprionate, triethylenetetraaminehexacetate, diethylenetriaminepentaacetate, dipicolinic acid, ethylenedicysteic acid (EDC), ethylenediamine-N,N'-bis(2-hydroxyphenylacetic acid) (EDDHA), glutamic acid diacetic acid (GLDA), hexadentateaminocarboxylate (HBED), polyethyleneimine, 1-hydroxydiphosphonate, aminotri(methylenephosphonic acid) (ATMP), nitrilotrimethylenephosphonate (NTP), ethylenediaminetetramethylenephosphonate, diethylenetriaminepentamethylenephosphonate (DTPMP), ethane-1-hydroxydiphosphonate (HEDP), 2-phosphonobutane-1,2,4-tricarboxylic acid, polvphosphoric acid, sodium tripolyphosphate, tetrasodium diphosphate, hexametaphosphoric acid, sodium metaphosphate, phosphonic acid and derivatives, Aminoalkylen-poly(alkylenphosphonic acid), aminotri(1-ethylphosphonic acid), , ethylenediaminetetra(1-ethylphosphonic acid), aminotri(1-propylphosphonic acid), aminotri(isopropylphosphonic acid), ethylenediaminetetra(methylenephosphonic acid) (EDTMP), 1,2-dihydroxy-3,5-disulfobenzene.

Aqueous Carrier

The hair care compositions can be in the form of pourable liquids (under ambient conditions). Such compositions will therefore typically comprise a carrier, which is present at a level of from about 40% to about 85%, alternatively from about 45% to about 80%, alternatively from about 50% to about 75% by weight of the hair care composition. The carrier may comprise water, or a miscible mixture of water and organic solvent, and in one aspect may comprise water with minimal or no significant concentrations of organic solvent, except as otherwise incidentally incorporated into the composition as minor ingredients of other essential or optional components.

The carrier useful the hair care compositions of the present invention may include water and water solutions of lower alkyl alcohols and polyhydric alcohols. The lower alkyl alcohols useful herein are monohydric alcohols having 1 to 6 carbons, in one aspect, ethanol and isopropanol. Exemplary polyhydric alcohols useful herein include propylene glycol, hexylene glycol, glycerin, and propane diol.

G. FOAM DISPENSER

The hair care composition described herein may be provided in a foam dispenser. The foam dispenser may be an aerosol foam dispenser. The aerosol foam dispenser may comprise a reservoir for holding the hair treatment composition. The reservoir may be made out of any suitable material selected from the group consisting of plastic, metal, alloy, laminate, and combinations thereof. And the reservoir may be for one-time use. The reservoir may be removable from the aerosol foam dispenser. Alternatively, the reservoir may be integrated with the aerosol foam dispenser. And there may be two or more reservoirs.

The foam dispenser may also be a mechanical foam dispenser. The mechanical foam dispenser described may be selected from the group consisting of squeeze foam dispensers, pump foam dispensers, other mechanical foam dispensers, and combinations thereof. The mechanical foam dispenser may be a squeeze foam dispenser. Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706).

The mechanical foam dispenser may comprise a reservoir for holding the hair treatment composition. The reservoir may be made out of any suitable material selected from the group consisting of plastic, metal, alloy, laminate, and combinations thereof. The reservoir may be a refillable reservoir such as a pour-in or screw-on reservoir, or the reservoir may be for one-time use. The reservoir may also be removable from the mechanical foam dispenser. Alternatively, the reservoir may be integrated with the mechanical foam dispenser. And there may be two or more reservoirs.

The reservoir may be comprised of a material selected from the group consisting of rigid materials, flexible

materials, and combinations thereof. The reservoir may be comprised of a rigid material if it does not collapse under external atmospheric pressure when it is subject to an interior partial vacuum.

H. PRODUCT FORM

The hair care compositions of the present invention may be presented in typical hair care formulations. They may be in the form of solutions, dispersion, emulsions, powders, talcs, encapsulated, spheres, spongers, solid dosage forms, foams, and other delivery mechanisms. The compositions the present invention may be hair tonics, leave-on hair products such as treatment, and styling products, rinse-off hair products such as shampoos and personal cleansing products, and treatment products; and any other form that may be applied to hair.

I. APPLICATOR

In the present invention, the hair care composition may be dispensed from an applicator for dispensing directly to the scalp area. Dispensing directly onto the scalp via a targeted delivery applicator enables deposition of the non diluted cleaning agents directly where the cleaning needs are highest. This also minimizes the risk of eye contact with the cleansing solution.

The applicator is attached or can be attached to a bottle containing the cleansing hair care composition. The applicator can consist of a base that holds or extends to a single or plurality of tines. The tines have openings that may be at the tip, the base or at any point between the tip and the base. These openings allows for the product to be distributed from the bottle directly onto the hair and/or scalp.

Alternatively, the applicator can also consist of brush-like bristles attached or extending from a base. In this case product would dispense from the base and the bristles would allow for product distribution via the combing or brushing motion.

Applicator and tine design and materials can also be optimized to enable scalp massage. In this case it would be beneficial for the tine or bristle geometry at the tips to be more rounded similar to the roller ball applicator used for eye creams. It may also be beneficial for materials to be smoother and softer; for example metal or metal-like finishes, "rubbery materials".

**Viscosity Measurement**

Shampoo viscosities can be measured on a 2.5 mL sample using a cone and plate Brookfield RS rheometer with cone C75-1 at 2 $s^{-1}$, 27°C at 3 mins.

In the present invention, wherein the surfactant composition alone with the surfactant soluble antidandruff agent has a viscosity of less than about 5000 cps at $2s^{-1}$ and is unable to be thickened above 5000 cps at $2s^{-1}$ with sodium chloride salt in the range of about 0.1% to 3%. Further in the present invention, wherein the surfactant composition alone with the surfactant soluble antidandruff agent has a viscosity of less than about 4000 cps at $2s^{-1}$ and is unable to be thickened above 4000 cps at $2s^{-1}$ with sodium chloride salt in the range of about 0.1% to 3% In the present invention, wherein the surfactant composition alone with the surfactant soluble antidandruff agent has a viscosity of less than about 3000 cps at $2s^{-1}$ and is unable to be thickened above 3000 cps at $2s^{-1}$ with sodium chloride salt in the range of about 0.1% to 3%. In the present invention, wherein the surfactant composition alone with the surfactant soluble antidandruff agent has a viscosity of less than about 2000 cps at $2s^{-1}$ and is unable to be thickened above 2000 cps at $2s^{-1}$ with sodium chloride salt in the range of about 0.1% to 3%.

**Measurement of % Transmittance (%T)**

Techniques for analysis of formation of complex coacervates are known in the art. One method to assess coacervate formation upon dilution for a transparent or translucent composition is to use a spectrophotometer to measure the percentage of light transmitted through the diluted sample (%T).. As percent light transmittance (%T) values measured of the dilution decrease, typically higher levels of coacervate are formed. Dilutions samples at various weight ratios of water to composition can be prepared, for example 2 parts of water to 1 part composition (2:1), or 7.5 parts of water to 1 part composition (7.5:1), or 16 parts of water to 1 part composition (16:1), or 34 parts of water to 1 part composition (34:1), and the %T measured for each dilution ratio sample. Examples of possible dilution ratios may include 2:1, 3:1, 5:1, 7.5:1, 11:1, 16:1, 24:1, or 34:1. By averaging the %T values for samples that span a range of dilution ratios, it is possible to simulate and ascertain how much coacervate a composition on average would form as a consumer applies the composition to wet hair, lathers, and then rinses it out. Average %T can be calculated by taking the numerical average of individual %T measurements for the following dilution ratios: 2:1, 3:1, 5:1, 7.5:1, 11:1, 16:1, 24:1, and 34:1.

%T can be measured using Ultra-Violet/Visible (UV/VI) spectrophotometry which determines the transmission of

UV/VIS light through a sample. A light wavelength of 600 nm has been shown to be adequate for characterizing the degree of light transmittance through a sample. Typically, it is best to follow the specific instructions relating to the specific spectrophotometer being used. In general, the procedure for measuring percent transmittance starts by setting the spectrophotometer to 600 nm. Then a calibration "blank" is run to calibrate the readout to 100 percent transmittance. A single test sample is then placed in a cuvette designed to fit the specific spectrophotometer and care is taken to insure no air bubbles are within the sample before the %T is measured by the spectrophotometer at 600 nm. Alternatively, multiple samples can be measured simultaneously by using a spectrophotometer such as the SpectraMax M-5 available from Molecular Devices. Multiple dilution samples can be prepared within a 96 well plate (VWR catalog# 82006-448) and then transferred to a 96 well visible flat bottom plate (Greiner part #655-001), ensuring that no air bubbles are within the sample. The flat bottom plate is placed within the SpectraMax M-5 and %T measured using the Software Pro v.5TM software available from Molecular Devices.

**Measurement of Anti-dandruff Agent Deposition**

Surfactant-soluble antidandruff agent, for example Octopirox, deposition in-vivo on scalp can be determined by ethanol extraction of the agent after the scalp has been treated with a surfactant-soluble agent containing cleansing composition and rinsed off. The concentration of agent in the extraction solvent or solution is measured by HPLC. Quantitation is made by reference to a standard curve. The concentration detected by HPLC is converted into an amount collected in grams by using the concentration multiplied by volume.

The percent agent deposited can be calculated using the following equation:

$$\%\ agent\ deposited = \frac{\dfrac{grams\ of\ agent\ deposited}{area\ of\ scalp\ extracted}}{\dfrac{(wt.\%\ agent\ in\ shampoo) \times (grams\ of\ shampoo\ applied)}{area\ of\ scalp\ treated}} \times 100\%$$

The deposition efficiency can be calculated using the following equation:

$$Deposition\ efficiency = \frac{\%\ agent\ deposited\ by\ example\ formula}{\%\ agent\ deposited\ by\ control\ formula}$$

Sample Calculation for % Piroctone Olamine deposited, where:

$$\text{Grams of agent deposited} = 1.8 \times 10^{-6} g$$

Area of scalp extracted = 1 $cm^2$
Wt % Piroctone Olamine in shampoo = 1.0%
Grams of shampoo applied = 5 $g$
Area of scalp treated = 300 $cm^2$

$$\%\ Piroctone\ Olamine\ deposited = \frac{\dfrac{1.8 \times 10^{-6} g}{1\ cm^2}}{\dfrac{(1.0\%) \times (5\ g\ )}{300\ cm^2}} \times 100\%$$

$$\%\ Piroctone\ Olamine\ deposited = 1.08\%$$

Sample calculation for deposition efficiency, where:

% Piroctone Olamine deposited by example formula = 9.30%
% Piroctone Olamine deposited by control formula = 1.08%

$$Deposition\ efficiency = \frac{9.30\%}{1.08\%}$$

$$Deposition\ efficiency = 8.6X$$

**Preparation of Shampoo Compositions**

The shampoo compositions are prepared by adding surfactants, anti-dandruff agents, perfume, viscosity modifiers, cationic polymers and the remainder of the water with ample agitation to ensure a homogenous mixture. The mixture can be heated to 50-75°C to speed the solubilization of the soluble agents, then cooled. Product pH may be adjusted as necessary to provide shampoo compositions of the present invention which are suitable for application to human hair and scalp, and may vary from about pH 4 to 9, or from about pH 4 to 6, or from about pH 4 to 5.5, or from about pH 4 to 5, based on the selection of particular detersive surfactants and/or other components.

**Non-limiting Examples**

The shampoo compositions illustrated in the following examples are prepared by conventional formulation and mixing methods. All exemplified amounts are listed as weight percents on an active basis and exclude minor materials such as diluents, preservatives, color solutions, imagery ingredients, botanicals, and so forth, unless otherwise specified. All percentages are based on weight unless otherwise specified.

RESULTS

| Ingredients | Examples, active wt% | |
|---|---|---|
| | 1 (control) | 2 (comparative example) |
| Water | q.s. | q.s. |
| Sodium Laureth-1 Sulfate (SLE1S) [1] | 15.0 | |
| Sodium Undecyl Sulfate [2] | | 15.0 |
| Piroctone Olamine [3] | 1.0 | 1.0 |
| Sodium Benzoate [4] | 0.25 | 0.25 |
| Tetrasodium EDTA [5] | 0.13 | 0.13 |
| Methylchloroisothiazolinone/ Methylisothiazolinone [6] | 5 ppm | 5 ppm |
| Citric Acid [7] | 0.60 | 0.54 |
| Fragrance | 0.85 | 0.85 |
| | | |
| Sodium Chloride [8] | 1.0 | 1.0 |
| Viscosity (cps) | 14390 | 0 |
| | | |
| Additional Sodium Chloride [8] (total Sodium Chloride) | +1.0 (2.0) | +2.0 (3.0) |
| Viscosity (cps) | 4177 | 0 |

1 Sodium Laureth-1 Sulfate at 26% active, supplier: P&G
2 Sodium Undecyl Sulfate at 70% active, supplier P&G
3 Octopirox, supplier: Clariant
4 Sodium Benzoate Dense NF/FCC, supplier: Emerald Performance Materials
5 Dissolvine 220-S at 84% active, supplier: Akzo Nobel
6 Kathon CG at 1.5% active, supplier: Rohm & Haas

(continued)

| Ingredients | | Examples, active wt% | |
|---|---|---|---|
| | | 1 (control) | 2 (comparative example) |
| Water | | q.s. | q.s. |
| 7 | Citric Acid Anhydrous, supplier: Archer Daniels Midland; level adjustable to achieve target pH | | |
| 8 | Sodium Chloride, supplier: Morton; level adjustable to achieve target viscosity | | |

| Ingredient | Examples, active wt% | |
|---|---|---|
| | 3 (control) | 4 (comparative example) |
| Water | q.s. | q.s. |
| Sodium Laureth-1 Sulfate (SLE1 S) [1] | 14.00 | - |
| Sodium Undecyl Sulfate [2] | - | 14.00 |
| Piroctone Olamine [3] | 1.00 | 1.00 |
| Sodium Benzoate [4] | 0.25 | 0.25 |
| Tetrasodium EDTA [5] | 0.13 | 0.13 |

(continued)

| Ingredient | Examples, active wt% | |
|---|---|---|
| | 3 (control) | 4 (comparative example) |
| Water | q.s. | q.s. |
| Methylchloroisothiazolinone/ Methylisothiazolinone 6 | 5 ppm | 5 ppm |
| Citric Acid 7 | 0.51 | 0.47 |
| | | |
| % Piroctone Olamine deposited | 1.1% | 1.9% |
| Deposition Efficiency (vs control) | 1.0X | 1.7X |

| | |
|---|---|
| 1 | Sodium Laureth-1 Sulfate at 26% active, supplier: P&G |
| 2 | Sodium Undecyl Sulfate at 70% active, supplier: P&G |
| 3 | Octopirox, supplier: Clariant |
| 4 | Sodium Benzoate Dense NF/FCC, supplier: Emerald Performance Materials |
| 5 | Dissolvine 220-S, supplier: Akzo Nobel |
| 6 | Kathon CG, supplier: Rohm & Haas |
| 7 | Citric Acid Anhydrous, supplier: Archer Daniels Midland; level adjustable to achieve target pH |

**Discussion of Results for Examples 1-4**

Sodium laureth-1 sulfate (SLE1S) is a surfactant that forms more stable micelles and as such forms elongated micelles, and shows an increase in viscosity upon the addition of sodium chloride. This increase in viscosity for SLE1S

upon sodium chloride addition is demonstrated in Example 1.

Sodium undecyl sulfate on the other hand is a surfactant that forms less stable micelles and as such does not form elongated micelles and therefore does not show an increase in viscosity upon the addition of sodium chloride. The lack of viscosity increase upon sodium chloride addition for Sodium undecyl sulfate is shown in Example 2.

A comparison of Examples 3 & 4 shows that Example 4, which contains sodium undecyl sulfate that forms less stable micelles, deposits Piroctone Olamine with 1.7X the deposition efficiency of Example 3, which contains sodium laureth-1 sulfate that forms more stable micelles.

| Ingredients | Examples, active wt% | | |
|---|---|---|---|
| | 5 (comparative example) | 6 (comparative example) | 7 (comparative example) |
| Water | q.s. | q.s. | q.s. |
| Sodium Decyl Sulfate [1] | 15.0 | | |
| Sodium Deceth-1 Sulfate [2] | | 15.0 | |
| Sodium Trideceth-2 Sulfate [3] | | | 15.0 |
| Piroctone Olamine [4] | 1.0 | 1.0 | 1.0 |
| Sodium Benzoate [5] | 0.25 | 0.25 | 0.25 |
| Tetrasodium EDTA [6] | 0.13 | 0.13 | 0.13 |
| Methylchloroisothiazolinone/ Methylisothiazolinone [7] | 5 ppm | 5 ppm | 5 ppm |
| Citric Acid [8] | 0.54 | 0.53 | 0.50 |
| Fragrance | 0.85 | 0.85 | 0.85 |
| | | | |
| Sodium Chloride [9] | 1.0 | 1.0 | 1.0 |
| Viscosity (cps) | 0 | 0 | 0 |
| | | | |
| Additional Sodium Chloride [11] (total Sodium Chloride) | +2.0 (3.0) | +2.0 (3.0) | +2.0 (3.0) |
| Viscosity (cps) | 0 | 0 | 0 |
| 1 | Sodium Decyl Sulfate at 70% active, supplier: P&G | | |
| 2 | Sodium Deceth-1 Sulfate at 70% active, supplier P&G | | |
| 3 | STEOL-TD 402-65 at 65% active, supplier: Stepan | | |
| 4 | Octopirox, supplier: Clariant | | |
| 5 | Sodium Benzoate Dense NF/FCC, supplier: Emerald Performance Materials | | |
| 6 | Dissolvine 220-S at 84% active, supplier: Akzo Nobel | | |
| 7 | Kathon CG at 1.5% active, supplier: Rohm & Haas | | |
| 8 | Citric Acid Anhydrous, supplier: Archer Daniels Midland; level adjustable to achieve target pH | | |
| 9 | Sodium Chloride, supplier: Morton; level adjustable to achieve target viscosity | | |

| Ingredients | Examples, active wt% | | | | |
|---|---|---|---|---|---|
| | 8 (comparative example) | 9 (comparative example) | 10 (comparative example) | 11 (comparative example) | |
| Water | q.s. | q.s. | q.s. | q.s. | |
| Sodium Laureth-1 Sulfate (SLE1S) [1] | 7.5 | 7.5 | 10.0 | | |
| Sodium Decyl Sulfate [2] | 7.5 | | 5.0 | | |
| Sodium Deceth-1 Sulfate [3] | | 7.5 | | 7.5 | |
| Sodium Trideceth-2 Sulfate [4] | | | | 7.5 | |
| Piroctone Olamine [5] | 1.0 | 1.0 | 1.0 | 1.0 | |
| Sodium Benzoate [6] | 0.25 | 0.25 | 0.25 | 0.25 | |
| Tetrasodium EDTA [7] | 0.13 | 0.13 | 0.13 | 0.13 | |
| Methylchloroisothiazolinone/ Methylisothiazolinone [8] | 5 ppm | 5 ppm | 5 ppm | 5 ppm | |
| Citric Acid [9] | 0.54 | 0.52 | 0.50 | 0.48 | |
| Fragrance | 0.85 | 0.85 | 0.85 | 0.85 | |
| | | | | | |
| Sodium Chloride [10] | 1.0 | 1.0 | 2.4 | 1.0 | |
| Viscosity (cps) | | 0 | 0 | 1034 | 0 |
| | | | | | |
| Additional Sodium Chloride [10] (total Sodium Chloride) | | +2.0 (3.0) | +2.0 (3.0) | +0.8 (3.2) | +2.0 (3.0) |
| Viscosity (cps) | | 0 | 646 | 1707 | 261 |

| | |
|---|---|
| 1 | Sodium Laureth-1 Sulfate at 26% active, supplier: P&G |
| 2 | Sodium Decyl Sulfate at 70% active, supplier P&G |
| 3 | Sodium Deceth-1 Sulfate at 70% active, supplier P&G |
| 4 | STEOL-TD 402-65 at 65% active, supplier: Stepan |
| 5 | Octopirox, supplier: Clariant |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Citric Acid Anhydrous, supplier: Archer Daniels Midland; level adjustable to achieve target pH |
| 10 | Sodium Chloride, supplier: Morton; level adjustable to achieve target viscosity |

**Discussion of Results for Examples 5-11**

Examples 5-7 are further examples of surfactant combinations that form less stable micelles and cannot be thickened with the addition of sodium chloride. Examples 8-10 show that when surfactants that form less stable micelles are combined with a surfactant (SLE1S) that forms more stable micelles, these compositions still cannot be thickened to a viscosity greater than about 2000 cps with sodium chloride. Example 11 shows that the combination of two surfactants that form less stable micelles forms a mixture that still cannot be thickened to a viscosity greater than 2000 cps with sodium chloride.

| Ingredients | Examples, active wt% | | | |
|---|---|---|---|---|
| | 12 (comparative example) | 13 (comparative example) | 14 (comparative example) | |
| Water | q.s. | q.s. | q.s. | |
| Sodium Laureth-1 Sulfate (SLE1S) [1] | 6.5 | 6.0 | 9.0 | |
| Sodium Decyl Sulfate [2] | | | | 4.0 |
| Sodium Deceth-1 Sulfate [3] | | 6.5 | 6.0 | |
| Cocamidopropyl Betaine (CAPB) [4] | | 1.0 | 1.5 | 1.0 |
| Cocamide MEA (CMEA) [5] | | 1.0 | 1.5 | 0.9 |
| Piroctone Olamine [6] | | 1.0 | 1.0 | 1.0 |
| Sodium Benzoate [7] | | 0.25 | 0.25 | 0.25 |
| Tetrasodium EDTA [8] | | 0.13 | 0.13 | 0.13 |
| Methylchloroisothiazolinone/ Methylisothiazolinone [9] | | 5 ppm | 5 ppm | 5 ppm |
| Citric Acid [10] | | 0.50 | 0.46 | 0.31 |
| Fragrance | | 0.85 | 0.85 | 0.85 |
| | | | | |
| Sodium Chloride [11] | | 1.0 | 1.0 | 1.0 |
| Viscosity (cps) | | 459 | 653 | 1319 |
| | | | | |
| Additional Sodium Chloride [11] (total Sodium Chloride) | | +2.0 (3.0) | +2.0 (3.0) | +0.9 (2.4) |
| Viscosity (cps) | | 241 | 347 | 0 |

1 Sodium Laureth-1 Sulfate at 26% active, supplier: P&G
2 Sodium Decyl Sulfate at 70% active, supplier: P&G
3 Sodium Deceth-1 Sulfate at 70% active, supplier: P&G
4 Tego Betain L 7 OK at 30% active, supplier: Evonik
5 Ninol Comf at 85% active, supplier: Stepan
6 Octopirox, supplier: Clariant
7 Sodium Benzoate Dense NF/FCC, supplier: Emerald Performance Materials
8 Dissolvine 220-S at 84% active, supplier: Akzo Nobel
9 Kathon CG at 1.5% active, supplier: Rohm & Haas
10 Citric Acid Anhydrous, supplier: Archer Daniels Midland; level adjustable to achieve target pH
11 Sodium Chloride, supplier: Morton; level adjustable to achieve target viscosity

**Discussion of Results for Examples 12-14**

Examples 12-14 show that when surfactants that form less stable micelles are combined with a surfactant (SLE1S) that forms stable micelles and also combined with co-surfactants (CAPB and CMEA) that typically increase viscosity, these mixtures still cannot be thickened with sodium chloride to a viscosity greater than 2000 cps.

| Ingredients | Examples, active wt% | | |
|---|---|---|---|
| | 15 (comparative example) | 16 (comparative example) | 17 |
| Sodium Laureth-1 Sulfate [1] | 7.5 | 7.5 | 7.5 |
| Sodium Laureth-3 Sulfate [2] | 7.5 | | |
| Sodium Decyl Sulfate [3] | | 7.5 | 7.5 |
| Piroctone Olamine [4] | 1.0 | 1.0 | 1.0 |
| Acrylates Copolymer [5] | | | 2.5 |
| Guar Hydroxypropyltrimonium Chloride [6] | 0.4 | 0.4 | 0.4 |
| Sodium Benzoate [7] | 0.25 | 0.25 | 0.25 |
| Tetrasodium EDTA [8] | 0.13 | 0.13 | 0.13 |

(continued)

| Ingredients | Examples, active wt% | | |
|---|---|---|---|
| | 15 (comparative example) | 16 (comparative example) | 17 |
| Methylchloroisothiazolinone/ Methylisothiazolinone [9] | 5 ppm | 5 ppm | 5 ppm |
| Citric Acid [10] | 0.53 | 0.85 | 0.32 |
| Fragrance | 1.0 | 1.0 | 1.0 |
| Sodium Chloride [11] | 0.25 | 0.25 | 0.26 |
| Water | q.s. | q.s. | q.s. |
| | | | |
| Average % T | 59% | 70% | 44% |

| | |
|---|---|
| 1 | Sodium Laureth-1 Sulfate at 26% active, supplier: P&G |
| 2 | Sodium Laureth-3 Sulfate at 28% active, supplier: P&G |
| 3 | Sodium Decyl Sulfate at 70% active, supplier: P&G |
| 4 | Octopirox, supplier: Clariant |
| 5 | Carbopol Aqua SF-1 at 30% active, supplier: Lubrizol |
| 6 | N-Hance BF-17, supplier: Ashland Specialty Ingredients |
| 7 | Sodium Benzoate Dense NF/FCC, supplier: Emerald Performance Materials |
| 8 | Dissolvine 220-S at 84% active, supplier: Akzo Nobel |
| 9 | Kathon CG at 1.5% active, supplier: Rohm & Haas |
| 10 | Citric Acid Anhydrous, supplier: Archer Daniels Midland; level adjustable to achieve target pH |
| 11 | Sodium Chloride, supplier: Morton; level adjustable to achieve target viscosity |

**Discussion of Results for Examples 15-17**

One method to assess coacervate formation upon dilution of a transparent or translucent composition is to measure the percentage of light transmitted through the diluted sample. By measuring the percent light transmittance (%T) of samples that span a range of water to composition dilution ratios and then averaging those %T values, it is possible to simulate and ascertain how much coacervate a composition would on average form as a consumer applies that composition to wet hair, lathers, and then rinses it out. The more coacervate that is formed, the less light is transmitted through the diluted sample and the lower the %T value.

Example 15 contains surfactants that form more stable micelles and as such, when combined with a cationic polymer such as guar hydroxypropyltrimonium chloride, forms a typical amount of coacervate upon dilution as observed in the average

%T of 59%. Example 16 contains a surfactant combination that forms less stable micelles such that when combined with guar hydroxypropytrimonium chloride only, this composition forms a lesser amount of coacervate upon dilution as observed in the average %T of 70%. Example 17 is an aspect of the present invention and contains a surfactant combination that forms less stable micelles in combination with both an anionic polymer (acrylates copolymer) and a cationic polymer (guar hydroxypropyltrimonium chloride). The composition of Example 17 upon dilution shows a surprising increase in coacervate formation as observed by the decrease in average %T from 70% in Example 16 to 44% in Example 17.

| Ingredients | Examples, active wt% | |
|---|---|---|
| | 18 (comparative example) | 19 |
| Sodium Laureth-1 Sulfate [1] | 7.5 | 7.5 |
| Sodium Decyl Sulfate [2] | 7.5 | 7.5 |
| Piroctone Olamine [3] | 1.0 | 1.0 |
| Acrylates Copolymer [4] | | 2.0 |
| Guar Hydroxypropyltrimonium Chloride [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.09 |
| Citric Acid [10] | 0.64 | 0.5 |
| Fragrance | 1.0 | 1.0 |
| Sodium Chloride [11] | 0.5 | 0.5 |
| Water | q. s. | q. s. |
| | | |
| Average %T | 72% | 52% |

1 Sodium Laureth-1 Sulfate at 26% active, supplier: P&G
2 Sodium Decyl Sulfate at 70% active, supplier: P&G
3 Octopirox, supplier: Clariant
4 Carbopol Aqua SF-1 at 30% active, supplier: Lubrizol
5 N-Hance 3196, supplier: Ashland
6 Sodium Benzoate Dense NF/FCC, supplier: Emerald Performance Materials
7 Dissolvine 220-S at 84% active, supplier: Akzo Nobel
8 Kathon CG at 1.5% active, supplier: Rohm & Haas
9 Sodium Hydroxide - Caustic Soda at 50% active, supplier: K.A. Steel Chemicals, Inc.; level adjustable as process aid or to achieve target pH
10 Citric Acid Anhydrous, supplier: Archer Daniels Midland; level adjustable to achieve target pH
11 Sodium Chloride, supplier: Morton; level adjustable to achieve target viscosity

| Ingredients | Examples, active wt% | |
|---|---|---|
| | 20 (comparative example) | 21 |
| Sodium Laureth-1 Sulfate [1] | 7.5 | 7.5 |
| Sodium Decyl Sulfate [2] | 7.5 | 7.5 |
| Piroctone Olamine [3] | 1.0 | 1.0 |
| Acrylates Copolymer [4] | | 2.0 |

(continued)

| Ingredients | Examples, active wt% | |
|---|---|---|
| | 20 (comparative example) | 21 |
| Polyquaternium-10 [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.06 |
| Citric Acid [10] | 0.5 | 0.46 |
| Fragrance | 1.0 | 1.0 |
| Sodium Chloride [11] | 0.5 | 0.5 |
| Water | q.s. | q.s. |
| | | |
| Average %T | 89% | 68% |

| | |
|---|---|
| 1 | Sodium Laureth-1 Sulfate at 26% active, supplier: P&G |
| 2 | Sodium Decyl Sulfate at 70% active, supplier: P&G |
| 3 | Octopirox, supplier: Clariant |
| 4 | Carbopol Aqua SF-1 at 30% active, supplier: Lubrizol |
| 5 | UCARE Polymer KG-30M, supplier: Dow Chemical |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Sodium Hydroxide - Caustic Soda at 50% active, supplier: K.A. Steel Chemicals, Inc.; level adjustable as process aid or to achieve target pH |
| 10 | Citric Acid Anhydrous, supplier: Archer Daniels Midland; level adjustable to achieve target pH |
| 11 | Sodium Chloride, supplier: Morton; level adjustable to achieve target viscosity |

**Discussion of Results for Examples 18-21**

Examples 19 and 21 are representative of compositions of the present invention and demonstrate an increase in coacervate formation upon dilution as evidenced by a decrease in average %T versus their respective comparative examples, Examples 18 and 20 which do not contain the anionic polymer (acrylates copolymer).

| Ingredients | Examples, active wt% | |
|---|---|---|
| | 22 (comparative example) | 23 |
| Sodium Deceth-1 Sulfate [1] | 7.5 | 7.5 |

(continued)

| Ingredients | Examples, active wt% | |
|---|---|---|
| | 22 (comparative example) | 23 |
| Sodium Trideceth-2 Sulfate [2] | 7.5 | 7.5 |
| Piroctone Olamine [3] | 1.0 | 1.0 |
| Acrylates Copolymer [4] | | 2.5 |
| Guar Hydroxypropyltrimonium Chloride [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.11 |
| Citric Acid [10] | 0.5 | 0.39 |
| Fragrance | 1.0 | 1.0 |

(continued)

| Ingredients | Examples, active wt% | |
|---|---|---|
| | 22 (comparative example) | 23 |
| Sodium Chloride [11] | 1.0 | 1.0 |
| Water | q.s. | q.s. |
| | | |
| Average % T | 77% | 65% |

| | |
|---|---|
| 1 | Sodium Deceth-1 Sulfate at 70% active, supplier P&G |
| 2 | STEOL-TD 402-65 at 65% active, supplier: Stepan |
| 3 | Octopirox, supplier: Clariant |
| 4 | Carbopol Aqua SF-1 at 30% active, supplier: Lubrizol |
| 5 | Jaguar C500, supplier: Solvay |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Sodium Hydroxide - Caustic Soda at 50% active, supplier: K.A. Steel Chemicals, Inc.; level adjustable as process aid or to achieve target pH |
| 10 | Citric Acid Anhydrous, supplier: Archer Daniels Midland; level adjustable to achieve target pH |
| 11 | Sodium Chloride, supplier: Morton; level adjustable to achieve target viscosity |

| Ingredients | Examples, active wt% | |
|---|---|---|
| | 24 (comparative example) | 25 |
| Sodium Deceth-1 Sulfate [1] | 7.5 | 7.5 |
| Sodium Trideceth-2 Sulfate [2] | 7.5 | 7.5 |
| Piroctone Olamine [3] | 1.0 | 1.0 |
| Acrylates Copolymer [4] | | 2.5 |

(continued)

| Ingredients | Examples, active wt% | |
|---|---|---|
| | 24 (comparative example) | 25 |
| Polyquaternium-10 [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.11 |
| Citric Acid [10] | 0.5 | 0.38 |
| Fragrance | 1.0 | 1.0 |
| Sodium Chloride [11] | 1.0 | 1.0 |
| Water | q.s. | q.s. |
| | | |
| Average % T | 67% | 54% |

| | |
|---|---|
| 1 | Sodium Deceth-1 Sulfate at 70% active, supplier P&G |
| 2 | STEOL-TD 402-65 at 65% active, supplier: Stepan |
| 3 | Octopirox, supplier: Clariant |
| 4 | Carbopol Aqua SF-1 at 30% active, supplier: Lubrizol |
| 5 | UCARE Polymer KG-30M, supplier: Dow Chemical |

(continued)

| Ingredients | Examples, active wt% | |
|---|---|---|
| | 24 (comparative example) | 25 |
| 6 | Sodium Benzoate Dense NF/FCC, supplier: Emerald Performance Materials | |
| 7 | Dissolvine 220-S at 84% active, supplier: Akzo Nobel | |
| 8 | Kathon CG at 1.5% active, supplier: Rohm & Haas | |
| 9 | Sodium Hydroxide - Caustic Soda at 50% active, supplier: K.A. Steel Chemicals, Inc.; level adjustable as process aid or to achieve target pH | |
| 10 | Citric Acid Anhydrous, supplier: Archer Daniels Midland; level adjustable to achieve target pH | |
| 11 | Sodium Chloride, supplier: Morton; level adjustable to achieve target viscosity | |

| Ingredients | Examples, active wt% | |
|---|---|---|
| | 26 (comparative example) | 27 |
| Sodium Deceth-1 Sulfate [1] | 7.5 | 7.5 |
| Sodium Trideceth-2 Sulfate [2] | 7.5 | 7.5 |
| Piroctone Olamine [3] | 1.0 | 1.0 |
| Acrylates Copolymer [4] | | 2.5 |
| Guar Hydroxypropyltrimonium Chloride [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.05 |
| Citric Acid [10] | 0.5 | 0.4 |
| Fragrance | 1.0 | 1.0 |
| Sodium Chloride [11] | 0.5 | 0.5 |
| Water | q.s. | q.s. |
| | | |
| Average %T | 49% | 35% |
| 1 | Sodium Deceth-1 Sulfate at 70% active, supplier P&G | |
| 2 | STEOL-TD 402-65 at 65% active, supplier: Stepan | |
| 3 | Octopirox, supplier: Clariant | |
| 4 | Carbopol Aqua SF-1 at 30% active, supplier: Lubrizol | |
| 5 | N-Hance BF-17, supplier: Ashland Specialty Ingredients | |
| 6 | Sodium Benzoate Dense NF/FCC, supplier: Emerald Performance Materials | |
| 7 | Dissolvine 220-S at 84% active, supplier: Akzo Nobel | |
| 8 | Kathon CG at 1.5% active, supplier: Rohm & Haas | |
| 9 | Sodium Hydroxide - Caustic Soda at 50% active, supplier: K.A. Steel Chemicals, Inc.; level adjustable as process aid or to achieve target pH | |
| 10 | Citric Acid Anhydrous, supplier: Archer Daniels Midland; level adjustable to achieve target pH | |
| 11 | Sodium Chloride, supplier: Morton; level adjustable to achieve target viscosity | |

**Discussion of Results for Examples 22-27**

Examples 23, 25, and 27 further exemplify compositions of the present invention. As such they demonstrate a surprising increase in coacervate formation upon dilution as evidenced by a decrease in average %T versus their respective comparative examples, Examples 22, 24, and 26, which do not contain anionic polymer (acrylates copolymer).

| Ingredients | Examples, active wt% | | | |
|---|---|---|---|---|
| | 28 (control) | 29 (comparative example A) | 30 (comparative example B) | 31 |
| Sodium Laureth-1 Sulfate [1] | 14.0 | | | |
| Sodium Deceth-1 Sulfate [1] | | 7.5 | 7.5 | 7.5 |
| Sodium Trideceth-2 Sulfate [2] | | 7.5 | 7.5 | 7.5 |
| Piroctone Olamine [3] | 1.0 | 1.0 | 1.0 | 1.0 |
| Acrylates Copolymer [4] | | | 2.5 | 2.5 |
| Guar Hydroxypropyltrimonium Chloride [5] | | 0.4 | | 0.4 |
| Sodium Benzoate [6] | 0.25 | 0.25 | 0.25 | 0.25 |
| Tetrasodium EDTA [7] | 0.13 | 0.13 | 0.13 | 0.13 |
| Methylchloroisothiazolinone/ Methylisothiazolinone [8] | 5 ppm | 5 ppm | 5 ppm | 5 ppm |
| Sodium Hydroxide [9] | | 0.05 | 0.14 | 0.08 |
| Citric Acid [10] | 0.55 | 0.56 | 0.37 | 0.32 |
| Fragrance | 1.0 | 1.0 | 1.0 | 1.0 |
| Sodium Chloride [11] | | 1.0 | 1.0 | 1.0 |
| Water | q.s. | q.s. | q.s. | q.s. |
| | | | | |
| % Piroctone Olamine deposition | 1.08% | 2.58% | 1.74% | 9.30% |
| Deposition efficiency (vs control) | 1.0X | - | - | 8.6X |
| Deposition efficiency (vs. comparative example A) | - | 1.0X | - | 3.6X |
| Deposition efficiency (vs. comparative example B) | - | - | 1.0X | 5.3X |

| | |
|---|---|
| 1 | Sodium Deceth-1 Sulfate at 70% active, supplier P&G |
| 2 | STEOL-TD 402-65 at 65% active, supplier: Stepan |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Sodium Hydroxide - Caustic Soda at 50% active, supplier: K.A. Steel Chemicals, Inc.; level adjustable as process aid or to achieve target pH |
| 10 | Citric Acid Anhydrous, supplier: Archer Daniels Midland; level adjustable to achieve target pH |

(continued)

| Ingredients | Examples, active wt% | | | |
|---|---|---|---|---|
| | 28 (control) | 29 (comparative example A) | 30 (comparative example B) | 31 |
| 11 | Sodium Chloride, supplier: Morton; level adjustable to achieve target viscosity | | | |

**Discussion of Results for Examples 28-31**

Example 31 exemplifies a composition of the present invention and demonstrates a surprising increase in % deposition and deposition efficiency of Piroctone Olamine (when compared to Example 28 as the control and Examples 29 & 30 as comparative examples) that is observed when cationic and anionic polymers are combined together as demonstrated in Example 31. From here on forth, all Piroctone Olamine deposition efficiencies will be calculated versus Example 28 as the control.

Examples 32-37 below further exemplify aspects of the present invention and show a surprising increase in % deposition and deposition efficiency versus Example 28 as the control.

| Ingredients | Examples, active wt% | | | |
|---|---|---|---|---|
| | 32 | 33 | 34 | 35 |
| Sodium Deceth-1 Sulfate [1] | 7.5 | 7.5 | 7.5 | 7.5 |
| Sodium Trideceth-2 Sulfate [2] | 7.5 | 7.5 | 7.5 | 7.5 |
| Piroctone Olamine [3] | 1.0 | 1.0 | 1.0 | 0.5 |
| Acrylates Copolymer [4] | 2.5 | 2.5 | 2.5 | 2.5 |
| Guar Hydroxypropyltrimonium Chloride [5] | 0.2 | | | 0.4 |

(continued)

| Ingredients | Examples, active wt% | | | |
|---|---|---|---|---|
| | 32 | 33 | 34 | 35 |
| Guar Hydroxypropyltrimonium Chloride [6] | | 0.4 | | |
| Polyquaternium-10 [7] | | | 0.4 | |
| Sodium Benzoate [8] | 0.25 | 0.25 | 0.25 | 0.25 |
| Tetrasodium EDTA [9] | 0.13 | 0.13 | 0.13 | 0.13 |
| Methylchloroisothiazolinone/ Methylisothiazolinone [10] | 5 ppm | 5 ppm | 5 ppm | 5 ppm |
| Sodium Hydroxide [11] | 0.11 | 0.11 | 0.11 | 0.13 |
| Citric Acid [12] | 0.44 | 0.39 | 0.38 | 0.51 |
| Fragrance | 1.0 | 1.0 | 1.0 | 1.0 |
| Sodium Chloride [13] | 1.0 | 1.0 | 1.0 | |
| Water | q.s. | q.s. | q.s. | q.s. |
| | | | | |

(continued)

| Ingredients | Examples, active wt% | | | |
|---|---|---|---|---|
| | 32 | 33 | 34 | 35 |
| % Piroctone Olamine deposition | 6.60% | 3.54% | 3.00% | 11.04% |
| Deposition efficiency (vs. control) | 6.1X | 3.3X | 2.8X | 10.2X |

| | |
|---|---|
| 1 | Sodium Deceth-1 Sulfate at 70% active, supplier P&G |
| 2 | STEOL-TD 402-65 at 65% active, supplier: Stepan |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | Jaguar C500, supplier: Solvay |
| 7 | UCARE Polymer KG-30M, supplier: Dow Chemical |
| 8 | Sodium Benzoate Dense NF/FCC, supplier: Emerald Performance Materials |
| 9 | Dissolvine 220-S at 84% active, supplier: Akzo Nobel |
| 10 | Kathon CG at 1.5% active, supplier: Rohm & Haas |
| 11 | Sodium Hydroxide - Caustic Soda at 50% active, supplier: K.A. Steel Chemicals, Inc.; level adjustable as process aid or to achieve target pH |
| 12 | Citric Acid Anhydrous, supplier: Archer Daniels Midland; level adjustable to achieve target pH |
| 13 | Sodium Chloride, supplier: Morton; level adjustable to achieve target viscosity |

| Ingredients | Examples, active wt% | |
|---|---|---|
| | 36 | 37 |
| Sodium Deceth-1 Sulfate [1] | 7.5 | 15 |
| Sodium Laureth-1 Sulfate [2] | 7.5 | |
| Piroctone Olamine [3] | 1 | 1 |
| Acrylates Copolymer [4] | 2.5 | 2.5 |
| Guar Hydroxypropyltrimonium Chloride [5] | 0.4 | 0.4 |
| Sodium Benzoate [6] | 0.25 | 0.25 |
| Tetrasodium EDTA [7] | 0.13 | 0.13 |
| Methylchloroisothiazolinone/ Methylisothiazolinone [8] | 5 ppm | 5 ppm |
| Sodium Hydroxide [9] | | 0.11 |
| Citric Acid [10] | 0.33 | 0.38 |
| Fragrance | 1.0 | 1.0 |
| Sodium Chloride [11] | 1.0 | 1.0 |

(continued)

| Ingredients | Examples, active wt% | |
|---|---|---|
| | 36 | 37 |
| Water | q.s. | q.s. |
| | | |
| % Piroctone Olamine deposition | 6.12% | 6.18% |
| Deposition efficiency (vs. control) | 5.7X | 5.7X |
| 1 | Sodium Deceth-1 Sulfate at 70% active, supplier P&G | |
| 2 | Sodium Laureth-1 Sulfate at 26% active, supplier: P&G | |
| 3 | Octopirox, supplier: Clariant | |
| 4 | Carbopol Aqua SF-1 at 30% active, supplier: Lubrizol | |
| 5 | N-Hance BF-17, supplier: Ashland Specialty Ingredients | |
| 6 | Sodium Benzoate Dense NF/FCC, supplier: Emerald Performance Materials | |
| 7 | Dissolvine 220-S at 84% active, supplier: Akzo Nobel | |
| 8 | Kathon CG at 1.5% active, supplier: Rohm & Haas | |
| 9 | Sodium Hydroxide - Caustic Soda at 50% active, supplier: K.A. Steel Chemicals, Inc.; level adjustable as process aid or to achieve target pH | |
| 10 | Citric Acid Anhydrous, supplier: Archer Daniels Midland; level adjustable to achieve target pH | |
| 11 | Sodium Chloride, supplier: Morton; level adjustable to achieve target viscosity | |

| Ingredients | Examples, active wt% | | |
|---|---|---|---|
| | 38 | 39 | 40 |
| Sodium Deceth-1 Sulfate [1] | 7.5 | 7.5 | 7.5 |
| Sodium Trideceth-2 Sulfate [2] | 7.5 | 7.5 | 7.5 |
| Piroctone Olamine [3] | 1 | 1 | 1 |
| Acrylates Copolymer [4] | 1 | | |
| Acrylates Copolymer [5] | | 2.5 | |
| Acrylates/C10-30 Alkyl Acrylate Crosspolymer [6] | | | 1 |
| Guar Hydroxypropyltrimonium Chloride [7] | 0.4 | 0.4 | 0.4 |
| Sodium Benzoate [8] | 0.25 | 0.25 | 0.25 |
| Tetrasodium EDTA [9] | 0.13 | 0.13 | 0.13 |
| Methylchloroisothiazolinone/ Methylisothiazolinone [10] | 5 ppm | 5 ppm | 5 ppm |
| Sodium Hydroxide [11] | | 0.16 | 0.07 |
| Citric Acid [12] | 0.48 | 0.4 | |
| Fragrance | 1.0 | 1.0 | 1.0 |
| Sodium Chloride [13] | 1.0 | 1.0 | 1.0 |
| Water | q.s. | q.s. | q.s. |
| | | | |
| % Piroctone Olamine deposition | 6.42% | 5.88% | 3.96% |

(continued)

| Ingredients | Examples, active wt% | | |
|---|---|---|---|
| | 38 | 39 | 40 |
| Deposition efficiency (vs. control) | 5.9X | 5.4X | 3.7X |

| | |
|---|---|
| 1 | Sodium Deceth-1 Sulfate at 70% active, supplier P&G |
| 2 | STEOL-TD 402-65 at 65% active, supplier: Stepan |
| 3 | Octopirox, supplier: Clariant |
| 4 | Carbopol Aqua SF-1 at 30% active, supplier: Lubrizol |
| 5 | Aculyn 33, supplier: Dow Chemical |
| 6 | Carbopol Ultrez 21, supplier: Lubrizol |
| 7 | N-Hance BF-17, supplier: Ashland Specialty Ingredients |
| 8 | Sodium Benzoate Dense NF/FCC, supplier: Emerald Performance Materials |
| 9 | Dissolvine 220-S at 84% active, supplier: Akzo Nobel |
| 10 | Kathon CG at 1.5% active, supplier: Rohm & Haas |
| 11 | Sodium Hydroxide - Caustic Soda at 50% active, supplier: K.A. Steel Chemicals, Inc.; level adjustable as process aid or to achieve target pH |
| 12 | Citric Acid Anhydrous, supplier: Archer Daniels Midland; level adjustable to achieve target pH |
| 13 | Sodium Chloride, supplier: Morton; level adjustable to achieve target viscosity |

**Discussion of Results for Examples 38-40**

Examples 38-40 are representative compositions of the present invention which contain varying levels and types of anionic polymers and demonstrate an increase in % deposition and deposition efficiency versus Example 28 as the control.

| Ingredients | Examples, active wt% | | | |
|---|---|---|---|---|
| | 41 (control) | 42 (comparative example) | 43 | |
| Sodium Laureth-1 Sulfate [1] | 14.0 | | | |
| Sodium Deceth-1 Sulfate [1] | | 7.5 | 7.5 | |
| Sodium Trideceth-2 Sulfate [2] | | 7.5 | 7.5 | |
| Climbazole [3] | | 1 | 1 | 1 |
| Acrylates Copolymer [4] | | | 2.5 | 2.5 |
| Guar Hydroxypropyltrimonium Chloride [5] | | | | 0.4 |
| Sodium Benzoate [6] | | 0.25 | 0.25 | 0.25 |
| Tetrasodium EDTA [7] | | 0.13 | 0.13 | 0.13 |
| Methylchloroisothiazolinone/ Methylisothiazolinone [8] | | 5 ppm | 5 ppm | 5 ppm |
| Sodium Hydroxide [9] | | 0.18 | 0.18 | 0.19 |
| Citric Acid [10] | | 0.38 | 0.38 | 0.39 |
| Fragrance | | 1.0 | 1.0 | 1.0 |

(continued)

| Ingredients | Examples, active wt% | | |
|---|---|---|---|
| | 41 (control) | 42 (comparative example) | 43 |
| Sodium Chloride [11] | 1.0 | 1.0 | 1.0 |
| Water | q.s. | q.s. | q.s. |
| | | | |
| % Climbazole deposition | 0.48% | 1.14% | 2.16% |
| Deposition efficiency (vs. control) | 1.0X | - | 4.5X |
| Deposition efficiency (vs. comparative example) | - | 1.0X | 1.9X |

| | |
|---|---|
| 1 | Sodium Deceth-1 Sulfate at 70% active, supplier P&G |
| 2 | STEOL-TD 402-65 at 65% active, supplier: Stepan |
| 3 | Crinipan AD, supplier: Symrise |
| 4 | Carbopol Aqua SF-1 at 30% active, supplier: Lubrizol |
| 5 | N-Hance BF-17, supplier: Ashland Specialty Ingredients |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Sodium Hydroxide - Caustic Soda at 50% active, supplier: K.A. Steel Chemicals, Inc.; level adjustable as process aid or to achieve target pH |
| 10 | Citric Acid Anhydrous, supplier: Archer Daniels Midland; level adjustable to achieve target pH |
| 11 | Sodium Chloride, supplier: Morton; level adjustable to achieve target viscosity |

**Discussion of Results for Examples 41-43**

Example 43 exemplifies a composition of the present invention and demonstrates that the surprising increase in % deposition and deposition efficiency (when compared to Example 41 as the control and Example 42 as a comparative example) is observed not only for Piroctone Olamine but is also observed for Climbazole.
The following examples are presented to further illustrate, but not to limit, the present invention:

| Ingredients | Examples, active wt% | | | |
|---|---|---|---|---|
| | 44 | 45 | 46 (comparative example) | 47 |
| Sodium Laureth-1 Sulfate (SLE1S) [1] | 7.5 | 7.5 | 7.5 | 11.0 |
| Sodium Deceth-1 Sulfate [2] | 7.5 | 7.5 | 7.5 | 1.0 |
| Piroctone Olamine [3] | 0.5 | 0.5 | 0.5 | 0.5 |
| Acrylates Copolymer [4] | 2.0 | 2.0 | 2.5 | 1.0 |
| Guar Hydroxypropyltrimonium Chloride [5] | 0.4 | | | |
| Polyquaternium-10 [6] | | 0.4 | 0.15 | |
| Guar Hydroxypropyltrimonium Chloride [7] | | | | 0.4 |

(continued)

| Ingredients | Examples, active wt% | | | |
|---|---|---|---|---|
| | 44 | 45 | 46 (comparative example) | 47 |
| Dimethiconol [8] | | | | 1.0 |
| Sodium Benzoate [9] | 0.25 | 0.25 | 0.25 | 0.25 |
| Tetrasodium EDTA [10] | 0.13 | 0.13 | 0.13 | 0.13 |
| Methylchloroisothiazolinone/ Methylisothiazolinone [11] | 5 ppm | 5 ppm | 5 ppm | 5 ppm |
| Sodium Hydroxide [12] | 0.13 | 0.07 | 0.24 | |
| Citric Acid [13] | 0.50 | 0.52 | 0.70 | 0.27 |
| Fragrance | 1.1 | 1.1 | 1.0 | 0.9 |
| Sodium Chloride [14] | 1.0 | 0.81 | 0.80 | 0.88 |
| Water | q.s. | q.s. | q.s. | q.s. |
| | | | | |
| pH | 6 | 6 | 6 | 6 |
| | | | | |
| % Piroctone Olamine deposition | 3.06% | 2.46% | 1.68% | 3.12 % |
| Deposition efficiency (vs. control) | 2.8X | 2.3X | 1.6X | 2.9X |

| | |
|---|---|
| 1 | Sodium Laureth-1 Sulfate at 26% active, supplier: P&G |
| 2 | Sodium Deceth-1 Sulfate at 70% active, supplier P&G |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | UCARE Polymer KG-30M, supplier: Dow Chemical |
| 7 | N-Hance 3196, supplier: Ashland Specialty Ingredients |
| 8 | Belsil DM5500 at 42% active, 100 nm particle size emulsion, supplier: Wacker |
| 9 | Sodium Benzoate Dense NF/FCC, supplier: Emerald Performance Materials |
| 10 | Dissolvine 220-S at 84% active, supplier: Akzo Nobel |
| 11 | Kathon CG at 1.5% active, supplier: Rohm & Haas |
| 12 | Sodium Hydroxide - Caustic Soda at 50% active, supplier: K.A. Steel Chemicals, Inc.; level adjustable as process aid or to achieve target pH |
| 13 | Citric Acid Anhydrous, supplier: Archer Daniels Midland; level adjustable to achieve target pH |
| 14 | Sodium Chloride, supplier: Morton; level adjustable to achieve target viscosity |

| Ingredients | Examples, active wt% | | | |
|---|---|---|---|---|
| | 48 | 49 (comparative example) | 50 (comparative example) | 51 (comparative example) |
| Sodium Laureth-1 Sulfate (SLE1S) [1] | 12.0 | 12.0 | 13.0 | 11.0 |
| Sodium Deceth-1 Sulfate [2] | 5.0 | 5.0 | 1.0 | 1.0 |
| Piroctone Olamine [3] | 0.5 | 0.5 | 0.5 | 0.5 |
| Acrylates Copolymer [4] | 2.0 | 0.4 | 0.4 | 0.4 |

(continued)

| Ingredients | Examples, active wt% | | | | |
|---|---|---|---|---|---|
| | 48 | 49 (comparative example) | 50 (comparative example) | 51 (comparative example) | |
| Guar Hydroxypropyltrimonium Chloride [5] | 0.4 | 0.4 | 0.4 | 0.4 | |
| Sodium Benzoate [6] | 0.25 | 0.25 | 0.25 | 0.25 | |
| Tetrasodium EDTA [7] | 0.13 | 0.13 | 0.13 | 0.13 | |
| Methylchloroisothiazolinone/ Methylisothiazolinone [8] | | 5 ppm | 5 ppm | 5 ppm | 5 ppm |
| Sodium Hydroxide [9] | | 0.10 | | | |
| Citric Acid [10] | | 0.48 | 0.47 | 0.46 | 0.35 |
| Fragrance | | 1.0 | 1.0 | 1.0 | 1.0 |
| Sodium Chloride [11] | | 0.24 | 2.0 | 1.2 | 1.5 |
| Water | | q.s. | q.s. | q.s. | q.s. |
| | | | | | |
| pH | | 6 | 6 | 6 | 6 |
| | | | | | |
| % Piroctone Olamine deposition | | 2.28% | 2.52% | 2.52% | 2.64% |
| Deposition efficiency (vs. control) | | 2.1X | 2.3X | 2.3X | 2.4X |

1 Sodium Laureth-1 Sulfate at 26% active, supplier: P&G
2 Sodium Deceth-1 Sulfate at 70% active, supplier P&G
3 Octopirox, supplier: Clariant
4 Carbopol Aqua SF-1 at 30% active, supplier: Lubrizol
5 N-Hance BF-17, supplier: Ashland Specialty Ingredients
6 Sodium Benzoate Dense NF/FCC, supplier: Emerald Performance Materials
7 Dissolvine 220-S at 84% active, supplier: Akzo Nobel
8 Kathon CG at 1.5% active, supplier: Rohm & Haas
9 Sodium Hydroxide - Caustic Soda at 50% active, supplier: K.A. Steel Chemicals, Inc.; level adjustable as process aid or to achieve target pH
10 Citric Acid Anhydrous, supplier: Archer Daniels Midland; level adjustable to achieve target pH
11 Sodium Chloride, supplier: Morton; level adjustable to achieve target viscosity

| Ingredients | Examples, active wt% | | | | |
|---|---|---|---|---|---|
| | 52 | 53 | 54 | 55 | |
| Sodium Laureth-1 Sulfate (SLE1S) [1] | 8.0 | 10.0 | 11.0 | 11.0 | |
| Sodium Deceth-1 Sulfate [2] | 4.0 | 4.0 | 4.0 | 6.0 | |
| Piroctone Olamine [3] | 0.5 | 0.5 | 0.5 | 0.5 | |
| Acrylates Copolymer [4] | | 1.6 | 1.6 | 1.6 | 2.0 |
| Guar Hydroxypropyltrimonium Chloride [5] | | 0.4 | 0.4 | 0.4 | 0.4 |
| Sodium Benzoate [6] | | 0.25 | 0.25 | 0.25 | 0.25 |

(continued)

| Ingredients | Examples, active wt% | | | |
|---|---|---|---|---|
| | 52 | 53 | 54 | 55 |
| Tetrasodium EDTA [7] | 0.13 | 0.13 | 0.13 | 0.13 |
| Methylchloroisothiazolinone/ Methylisothiazolinone [8] | 5 ppm | 5 ppm | 5 ppm | 5 ppm |
| Sodium Hydroxide [9] | 0.13 | 0.08 | 0.08 | 0.12 |
| Citric Acid [10] | 0.43 | 0.36 | 0.49 | 0.49 |
| Fragrance | 1.0 | 1.0 | 1.0 | 1.0 |
| Sodium Chloride [11] | 1.2 | 0.91 | 0.46 | |
| Water | q.s. | q.s. | q.s. | q.s. |
| | | | | |
| pH | 6 | 6 | 6 | 6 |
| | | | | |
| % Piroctone Olamine deposition | 3.36% | 2.76% | 3.48% | 2.64% |
| Deposition efficiency (vs. control) | 3.1X | 2.6X | 3.2X | 2.4X |

| | |
|---|---|
| 1 | Sodium Laureth-1 Sulfate at 26% active, supplier: P&G |
| 2 | Sodium Deceth-1 Sulfate at 70% active, supplier P&G |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | Sodium Benzoate Dense NF/FCC, supplier: Emerald Performance Materials |
| 7 | Dissolvine 220-S at 84% active, supplier: Akzo Nobel |
| 8 | Kathon CG at 1.5% active, supplier: Rohm & Haas |
| 9 | Sodium Hydroxide - Caustic Soda at 50% active, supplier: K.A. Steel Chemicals, Inc.; level adjustable as process aid or to achieve target pH |
| 10 | Citric Acid Anhydrous, supplier: Archer Daniels Midland; level adjustable to achieve target pH |
| 11 | Sodium Chloride, supplier: Morton; level adjustable to achieve target viscosity |

| Ingredients | Examples, active wt% | | | |
|---|---|---|---|---|
| | 56 | 57 | 58 | 59 |
| Sodium Laureth-1 Sulfate (SLE1S) [1] | 7.5 | 7.5 | 7.5 | 7.5 |
| Sodium Deceth-1 Sulfate [2] | 7.5 | 7.5 | 7.5 | 7.5 |
| Piroctone Olamine [3] | 0.5 | 0.5 | 0.5 | 0.5 |
| Acrylates Copolymer [4] | 2.0 | 1.8 | 1.8 | 1.8 |
| Guar Hydroxypropyltrimonium Chloride [5] | 0.4 | 0.4 | 0.4 | |

(continued)

| Ingredients | | Examples, active wt% | | |
|---|---|---|---|---|
| Polyquaternium-10 [6] | | | | 0.4 |
| Gel Network [7] | | 25.0 | 16.7 | 16.7 |
| Dimethiconol [8] | 1.0 | 2.5 | 1.0 | 1.0 |
| Glycerin [9] | | 5.0 | | |
| Sodium Benzoate [10] | 0.25 | 0.25 | 0.25 | 0.25 |
| Tetrasodium EDTA [11] | 0.13 | 0.13 | 0.13 | 0.13 |
| Methylchloroisothiazolinone/ Methylisothiazolinone [12] | 5 ppm | 5 ppm | 5 ppm | 5 ppm |
| Sodium Hydroxide [13] | 0.09 | 0.08 | 0.07 | 0.08 |
| Citric Acid [14] | 0.38 | 0.54 | 0.18 | 0.49 |
| Fragrance | 1.1 | 1.1 | 1.1 | 1.1 |
| Sodium Chloride [15] | 0.69 | | 0.17 | 0.14 |
| Water | q.s. | q.s. | q.s. | q.s. |
| | | | | |
| % Piroctone Olamine deposition | 2.88% | 3.48% | 3.12% | 1.92% |
| Deposition efficiency (vs. control) | 2.7X | 3.2X | 2.9X | 1.8X |

| | |
|---|---|
| 1 | Sodium Laureth-1 Sulfate at 26% active, supplier: P&G |
| 2 | Sodium Deceth-1 Sulfate at 70% active, supplier P&G |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | UCARE Polymer KG-30M, supplier: Dow Chemical |
| 7 | Gel Network with Fatty Alcohol; See Composition in table below. The water is heated to about 74°C and the Cetyl Alcohol, Stearyl Alcohol, and the SLES Surfactant are added to it. After incorporation, this mixture is passed through a heat exchanger where it is cooled to about 35°C. As a result of this cooling step, the Fatty Alcohols and surfactant crystallize to form a crystalline gel network. |
| 8 | Belsil DM5500 at 42% active, 100 nm particle size emulsion, supplier: Wacker |
| 9 | Glycerine 99.7% USP, supplier: P&G Chemicals |
| 10 | Sodium Benzoate Dense NF/FCC, supplier: Emerald Performance Materials |
| 11 | Dissolvine 220-S at 84% active, supplier: Akzo Nobel |
| 12 | Kathon CG at 1.5% active, supplier: Rohm & Haas |
| 13 | Sodium Hydroxide - Caustic Soda at 50% active, supplier: K.A. Steel Chemicals, Inc.; level adjustable as process aid or to achieve target pH |
| 14 | Citric Acid Anhydrous, supplier: Archer Daniels Midland; level adjustable to achieve target pH |
| 15 | Sodium Chloride, supplier: Morton; level adjustable to achieve target viscosity |

Gel Network with Fatty Alcohol Composition

| Ingredient | As added wt% |
|---|---|
| Water | 45.66% |
| Cetyl Alcohol | 4.29% |
| Steary Alcohol | 7.71% |
| Sodium laureth-1 sulfate (26% Active) | 42.31% |
| Methylchloroisothiazolinone/ Methylisothiazolinone, Kathon CG | 0.03% |

| Ingredients | Examples, active wt% | | | |
|---|---|---|---|---|
| | 60 (comparative example) | 61 | 62 (comparative example) | 63 |
| Sodium Laureth-1 Sulfate (SLE1S) [1] | 13.0 | 12.0 | 11.0 | 11.0 |
| Sodium Deceth-1 Sulfate [2] | 1.0 | | 1.0 | 1.0 |
| Cocamide MEA [3] | 0.5 | | | |
| Piroctone Olamine [4] | 0.5 | 0.5 | 0.5 | 0.5 |
| Acrylates Copolymer [5] | 0.7 | 1.0 | 1.0 | 1.0 |
| Guar Hydroxypropyltrimonium Chloride [6] | | 0.4 | 0.1 | 0.4 |

| | | | | |
|---|---|---|---|---|
| Polyquaternium-10 [7] | 0.4 | | | |
| Glycol Distearate [8] | 0.5 | 0.75 | | |
| Bis-Aminopropyl Dimethicone [9a] | 1.5 | | | |
| PEG-10 Dimethicone [9b] | 0.08 | | | |
| Trideceth-3 [9c] | 0.18 | | | |
| Laureth-9 [9d] | 0.16 | | | |
| Hydrochloric Acid [9e] | Up to 1% | | | |
| Dimethiconol [10] | | 1.0 | 1.0 | 1.0 |
| Sodium Benzoate [11] | 0.25 | 0.25 | 0.25 | 0.25 |
| Tetrasodium EDTA [12] | 0.13 | 0.13 | 0.13 | 0.13 |
| Methylchloroisothiazolinone/ Methylisothiazolinone [13] | 5 ppm | 5 ppm | 5 ppm | 5 ppm |
| Sodium Hydroxide [14] | | 0.17 | 0.17 | 0.04 |
| Citric Acid [15] | 0.30 | 1.1 | 0.54 | 0.63 |
| Fragrance | 1.0 | 1.0 | 1.0 | 0.9 |
| Sodium Chloride [16] | 0.04 | 0.15 | 1.8 | 0.67 |
| Water | q.s. | q.s. | q.s. | q.s. |
| | | | | |
| pH | 5 | 4.5 | 6 | 5 |
| | | | | |
| % Piroctone Olamine deposition | 2.88% | 1.56% | 1.56% | 3.96 % |
| Deposition efficiency (vs. control) | 2.7X | 1.4X | 1.4X | 3.7X |

1 Sodium Laureth-1 Sulfate at 26% active, supplier: P&G

2 Sodium Deceth-1 Sulfate at 70% active, supplier P&G

3 Ninol Comf at 85% active, supplier: Stepan

4 Octopirox, supplier: Clariant

5 Carbopol Aqua SF-1 at 30% active, supplier: Lubrizol

6 N-Hance BF-17, supplier: Ashland Specialty Ingredients

7 UCARE Polymer KG-30M, supplier: Dow Chemical

8 EGDS Purified, supplier: Evonik Goldschmidt Corporation

| | |
|---|---|
| 9 | Bis-Aminopropyl Dimethicone Emulsion having an average silicone particle size of about 50 nm to about 550 nm<br>The Bis-aminopropyl dimethicone (9a) is emulsified separately using various emulsifiers where Hydrochloric acid (9e) can be added to adjust the emulsion pH as needed and the completed emulsion is subsequently added to the composition. A non-limiting selection of emulsifiers (9b-d) are shown herein.<br>9a: Y-14945 Amino Fluid, supplier: Momentive Performance Materials<br>9b: KF-6017, supplier: Shin-Etsu Chemical Co., Ltd.<br>9c: Lutensol TDA 3, supplier: BASF Corporation<br>9d: Chemal LA-9, supplier: PCC Chemax, Inc.<br>9e: Muriatic Acid acid, 20% active, supplier: Hydrite Chemical Co. |
| 10 | Belsil DM5500 at 42% active, supplier: Wacker |
| 11 | Sodium Benzoate Dense NF/FCC, supplier: Emerald Performance Materials |
| 12 | Dissolvine 220-S at 84% active, supplier: Akzo Nobel |
| 13 | Kathon CG at 1.5% active, supplier: Rohm & Haas |
| 14 | Sodium Hydroxide – Caustic Soda at 50% active, supplier: K.A. Steel Chemicals, Inc.; level adjustable as process aid or to achieve target pH |
| 15 | Citric Acid Anhydrous, supplier: Archer Daniels Midland; level adjustable to achieve target pH |
| 16 | Sodium Chloride, supplier: Morton; level adjustable to achieve target viscosity |

| Ingredients | Examples, active wt% | | | |
|---|---|---|---|---|
| | 64 | 65 (comparative example) | 66 | 67 (comparative example) |
| Sodium Laureth-1 Sulfate (SLE1S) [1] | 11.0 | 14.0 | 12.0 | 12.0 |
| Sodium Deceth-1 Sulfate [2] | 1.0 | | | |
| Cocamide MEA [3] | | 0.5 | | 0.5 |
| Piroctone Olamine [4] | 0.5 | 0.6 | 0.6 | 0.5 |
| Acrylates Copolymer [5] | 1.0 | 0.7 | 1.0 | 0.7 |
| Guar Hydroxypropyltri-monium Chloride [6] | 0.4 | 0.4 | 0.4 | 0.4 |
| Glycol Distearate [7] | 0.75 | 0.5 | 0.75 | 0.5 |
| Bis-Aminopropyl Dimethicone [8a] | | 2.0 | | |
| PEG-10 Dimethicone [8b] | | 0.1 | | |

(continued)

| Ingredients | Examples, active wt% | | | |
|---|---|---|---|---|
| | 64 | 65 (comparative example) | 66 | 67 (comparative example) |
| Trideceth-3 [8c] | | 0.24 | | |
| Laureth-9 [8d] | | 0.21 | | |
| Hydrochloric Acid [8e] | | Up to 1% | | |
| Dimethiconol [9] | 1.0 | | 1.0 | 1.0 |
| Sodium Benzoate [10] | 0.25 | 0.25 | 0.25 | 0.25 |
| Tetrasodium EDTA [11] | 0.13 | 0.13 | 0.13 | 0.13 |
| Methylchloroisothiazolinone/ Methylisothiazolinone [12] | 5 ppm | 5 ppm | 5 ppm | 5 ppm |
| Sodium Hydroxide [13] | 0.04 | | 0.04 | |
| Citric Acid [14] | 0.34 | 0.65 | 0.85 | 0.60 |
| Fragrance | 1.0 | 1.0 | 0.9 | 1.0 |
| Sodium Chloride [15] | 1.1 | | 0.31 | 0.29 |
| Water | q.s. | q.s. | q.s. | q.s. |
| | | | | |
| pH | 6 | 5 | 4.5 | 5 |
| | | | | |
| % Piroctone Olamine deposition | 2.40% | 3.20% | 3.50% | 3.36% |
| Deposition efficiency (vs. control) | 2.2X | 3.0X | 3.2X | 3.1X |

| | |
|---|---|
| 1 | Sodium Laureth-1 Sulfate at 26% active, supplier: P&G |
| 2 | Sodium Deceth-1 Sulfate at 70% active, supplier P&G |
| 3 | Ninol Comf at 85% active, supplier: Stepan |
| 4 | Octopirox, supplier: Clariant |
| 5 | Carbopol Aqua SF-1 at 30% active, supplier: Lubrizol |
| 6 | N-Hance BF-17, supplier: Ashland Specialty Ingredients |
| 7 | EGDS Purified, supplier: Evonik Goldschmidt Corporation |
| 8 | Bis-Aminopropyl Dimethicone Emulsion having an average silicone particle size of about 50 nm to about 550 nm<br>The Bis-aminopropyl dimethicone (8a) is emulsified separately using various emulsifiers where Hydrochloric acid (8e) can be added to adjust the emulsion pH as needed and the completed emulsion is subsequently added to the composition. A non-limiting selection of emulsifiers (8b-d) are shown herein.<br>8a: Y-14945 Amino Fluid, supplier: Momentive Performance Materials<br>8b: KF-6017, supplier: Shin-Etsu Chemical Co., Ltd.<br>8c: Lutensol TDA 3, supplier: BASF Corporation<br>8d: Chemal LA-9, supplier: PCC Chemax, Inc. |

(continued)

| Ingredients | | Examples, active wt% | | | |
|---|---|---|---|---|---|
| | | 64 | 65 (comparative example) | 66 | 67 (comparative example) |
| | 8e: Muriatic Acid acid, 20% active, supplier: Hydrite Chemical Co. | | | | |
| 9 | Belsil DM5500 at 42% active, supplier: Wacker | | | | |
| 10 | Sodium Benzoate Dense NF/FCC, supplier: Emerald Performance Materials | | | | |
| 11 | Dissolvine 220-S at 84% active, supplier: Akzo Nobel | | | | |
| 12 | Kathon CG at 1.5% active, supplier: Rohm & Haas | | | | |
| 13 | Sodium Hydroxide - Caustic Soda at 50% active, supplier: K.A. Steel Chemicals, Inc.; level adjustable as process aid or to achieve target pH | | | | |
| 14 | Citric Acid Anhydrous, supplier: Archer Daniels Midland; level adjustable to achieve target pH | | | | |
| 15 | Sodium Chloride, supplier: Morton; level adjustable to achieve target viscosity | | | | |

| Ingredients | Examples, active wt% | | | |
|---|---|---|---|---|
| | 68 (comparative example) | 69 (comparative example) | 70 (comparative example) | 71 |
| Sodium Laureth-1 Sulfate (SLE1S) [1] | 11.0 | 12.0 | 10.0 | 7.5 |
| Sodium Deceth-1 Sulfate [2] | 1.0 | 5.0 | 4.0 | 7.5 |
| Piroctone Olamine [3] | 0.5 | 0.5 | 0.5 | 0.5 |
| Acrylates Copolymer [4] | 0.7 | 0.7 | 1.0 | 1.8 |
| Guar Hydroxypropyltrimonium Chloride [5] | 0.4 | 0.3 | 0.1 | |
| Polyquaternium-10 [6] | | | | 0.4 |
| Bis-Aminopropyl Dimethicone [7a] | 2.0 | 1.5 | 1.0 | 1.0 |
| Glycerin [7b] | | | | 0.01 |
| PEG-10 Dimethicone [7c] | 0.1 | | | |
| PEG-9 Polydimethylsiloxyethyl Dimethicone [7d] | | 0.11 | 0.01 | |
| Trideceth-3 [7e] | 0.24 | | 0.16 | 2.0 |
| Decyl Glucoside [7f] | | | 0.07 | 0.11 |
| Laureth-9 [7g] | 0.21 | | 0.01 | |
| C11-15 Pareth-5 [7h] | | 0.23 | | |
| C11-15 Pareth-3 [7i] | | 0.11 | | |

(continued)

| Ingredients | Examples, active wt% | | | |
|---|---|---|---|---|
| | 68 (comparative example) | 69 (comparative example) | 70 (comparative example) | 71 |
| C11-15 Pareth-12 [7j] | | 0.15 | | |
| Hydrochloric Acid [7k] | Up to 1% | Up to 1% | Up to 1% | Up to 1% |
| Sodium Benzoate [8] | 0.25 | 0.25 | 0.25 | 0.25 |
| Tetrasodium EDTA [9] | 0.13 | 0.13 | 0.13 | 0.13 |
| Methylchloroisothiazolinone/ Methylisothiazolinone [11] | 5 ppm | 5 ppm | 5 ppm | 5 ppm |
| Sodium Hydroxide [11] | Up to 1% | Up to 1% | Up to 1% | Up to 1% |
| Citric Acid [12] | Up to 2% | Up to 2% | Up to 2% | Up to 2% |
| Fragrance | 1.1 | 1.1 | 1.1 | 1.1 |
| Sodium Chloride [13] | Up to 3% | Up to 3% | Up to 3% | Up to 3% |
| Water | q.s. | q.s. | q.s. | q.s. |
| | | | | |
| pH | 6.0 | 5.0 | 4.5 | 5.5 |

| | |
|---|---|
| 1 | Sodium Laureth-1 Sulfate at 26% active, supplier: P&G |
| 2 | Sodium Deceth-1 Sulfate at 70% active, supplier P&G |
| 3 | Octopirox, supplier: Clariant |
| 4 | Carbopol Aqua SF-1 at 30% active, supplier: Lubrizol |
| 5 | N-Hance BF-17, supplier: Ashland Specialty Ingredients |
| 6 | UCARE Polymer KG-30M, supplier: Dow Chemical |

(continued)

| Ingredients | | Examples, active wt% | | | |
|---|---|---|---|---|---|
| | | 68 (comparative example) | 69 (comparative example) | 70 (comparative example) | 71 |
| 7 | Emulsion of Bis-Aminopropyl Dimethicone having an average silicone particle size of about 50 nm to about 550 nm; or having an average particle size of about 1.0 to about 10 microns<br>The Bis-aminopropyl dimethicone (7a) is emulsified separately using various emulsifiers where Hydrochloric acid (7k) can be added to adjust the emulsion pH as needed and the completed emulsion is subsequently added to the composition. A non-limiting selection emulsifiers (7b-7j) are shown herein.<br>7a: Y-14945 Amino Fluid, supplier: Momentive Performance Materials<br>7b: Glycerine 99.7% USP, supplier: P&G Chemicals<br>7c: KF-6017, supplier: Shin-Etsu Chemical Co., Ltd.<br>7d: KF-6028, supplier: Shin-Etsu Chemical Co., Ltd.<br>7e: Lutensol TDA 3, supplier: BASF Corporation<br>7f: Plantaren 2000 N UP, 50% active, supplier: BASF Corporation<br>7g: Chemal LA-9, supplier: PCC Chemax, Inc.<br>7h: Tergitol 15-S-5, supplier: Dow Chemical Co.<br>7i: Tergitol 15-S-3; supplier: Dow Chemical Co.<br>7j: Tergitol 15-S-12; supplier: Dow Chemical Co.<br>7k: Muriatic Acid acid, 20% active, supplier: Hydrite Chemical Co. | of | | | |
| 8 | Sodium Benzoate Dense NF/FCC, supplier: Emerald Performance Materials | | | | |

(continued)

| Ingredients | | Examples, active wt% | | | |
|---|---|---|---|---|---|
| | | 68 (comparative example) | 69 (comparative example) | 70 (comparative example) | 71 |
| 9 | Dissolvine 220-S at 84% active, supplier: Akzo Nobel | | | | |
| 10 | Kathon CG at 1.5% active, supplier: Rohm & Haas | | | | |
| 11 | Sodium Hydroxide - Caustic Soda at 50% active, supplier: K.A. Steel Chemicals, Inc.; adjustable as process aid or to achieve target pH | level | | | |
| 12 | Citric Acid Anhydrous, supplier: Archer Daniels Midland; level adjustable to achieve target pH | | | | |
| 13 | Sodium Chloride, supplier: Morton; level adjustable to achieve target viscosity | | | | |

| Ingredients | Examples, active wt% | | |
|---|---|---|---|
| | 72 | 73 (comparative example) | 74 (comparative example) |
| Sodium Laureth-1 Sulfate (SLE1S) [1] | 5.0 | | 10.0 |
| Sodium Undecyl Sulfate [2] | 6.0 | | |
| Sodium Undeceth-1 Sulfate [3] | | 8.0 | |
| Sodium Deceth-1 Sulfate [4] | | | 5.0 |
| Sodium Trideceth-2 Sulfate [5] | 1.0 | 6.0 | |
| Cocamidopropyl Betaine [6] | | 1.0 | |
| Piroctone Olamine [7] | 0.25 | 0.75 | 0.5 |
| Ammonium Acryloyldimethyltaurate/VP Copolymer [8] | 1.2 | | |
| Sodium Carboxymethylcellulose [9] | | 4.0 | |
| Poly(methyl methacrylate/-methacrylic acid) [90:10 monomer ratio] [10] | | | 3.1 |
| Guar Hydroxypropyltrimonium Chloride [11] | 0.2 | | |
| Guar Hydroxypropyltrimonium Chloride [12] | | | 0.3 |
| Polyquaternium-10 [13] | | 0.4 | |
| Polyquaternium-10 [14] | | | 0.2 |
| Polyquaternium-6 [15] | | 0.2 | |

(continued)

| Ingredients | Examples, active wt% | | | |
|---|---|---|---|---|
| | 72 | 73 (comparative example) | 74 (comparative example) | |
| Hydroxypropylguar Hydroxypropyltrimonium Chloride [16] | 0.1 | | | |
| Gel Network [17] | 12.5 | | 25.0 | |
| Dimethiconol [18] | 2.0 | | | |
| Linoleamidopropyl PG-Dimonium Chloride Phosphate [19] | 0.5 | 0.25 | | |
| Glycerin [20] | | 5.0 | | |
| Sodium Benzoate [21] | 0.25 | 0.25 | 0.25 | |
| Tetrasodium EDTA [22] | 0.13 | 0.13 | 0.13 | |
| Methylchloroisothiazolinone/ Methylisothiazolinone [23] | 5 ppm | 5 ppm | 5 ppm | |
| Sodium Hydroxide [24] | Up to 1% | Up to 1% | Up to 1% | |
| Citric Acid [25] | | Up to 2% | Up to 2% | Up to 2% |
| Fragrance | | 1.0 | 1.0 | 1.0 |
| Sodium Chloride [26] | | Up to 3% | Up to 3% | Up to 3% |
| Water | | q.s. | q.s. | q.s. |
| | | | | |
| pH | | 5.5 | 6.5 | 6.0 |

| | |
|---|---|
| 1 | Sodium Laureth-1 Sulfate at 26% active, supplier: P&G |
| 2 | Sodium Undecyl Suflate at 70% active, supplier: P&G |
| 3 | Sodium Undeceth-1 Suflate at 70% active, supplier: P&G |
| 4 | Sodium Deceth-1 Sulfate at 70% active, supplier P&G |
| 5 | STEOL-TD 402-65 at 65% active, supplier: Stepan |
| 6 | Tego Betain L 7 OK at 30% active, supplier: Evonik |
| 7 | Octopirox, supplier: Clariant |
| 8 | Aristoflex AVC, supplier: Clariant |
| 9 | Sodium Carboxymethylcellulose, supplier: Dow Chemical |
| 10 | Poly(methyl methacrylate/methacrylic acid) [90:10 monomer ratio], supplier: Polysciences, Inc. |
| 11 | Jaguar Excel, supplier: Solvay |
| 12 | N-Hance BF-13, supplier: Ashland Specialty Ingredients |
| 13 | UCARE Polymer JR30M, supplier: Dow Chemical |
| 14 | UCARE Polymer LR-30M, supplier: Dow Chemical |
| 15 | Mirapol 100 at 31.5% active, supplier: Solvay |
| 16 | Jaguar C-2000, supplier: Solvay |
| 17 | Gel Network with Fatty Acid; See Composition in table below. About 20% of the water is heated to about 74°C and the Cetyl Alcohol, Stearyl Alcohol, and the Stearic acid added to it. After incorporation, this mixture pH is adjusted with NaOH as needed to achieve the target pH of 6-8 and is passed through a mill and heat exchanger where it is cooled to about 35°C. As a result of this cooling step, the fatty acid, additional fatty amphiphile and the water form a crystalline gel network. |
| 18 | Belsil DM5500 at 42% active, supplier: Wacker |

(continued)

| Ingredients | Examples, active wt% | | |
|---|---|---|---|
| | 72 | 73 (comparative example) | 74 (comparative example) |
| 19 | Arlasilk EFA at 30% active, supplier: Croda | | |
| 20 | Glycerine 99.7% USP, supplier: P&G Chemicals | | |
| 21 | Sodium Benzoate Dense NF/FCC, supplier: Emerald Performance Materials | | |
| 22 | Dissolvine 220-S at 84% active, supplier: Akzo Nobel | | |
| 23 | Kathon CG at 1.5% active, supplier: Rohm & Haas | | |
| 24 | Sodium Hydroxide - Caustic Soda at 50% active, supplier: K.A. Steel Chemicals, Inc.; level adjustable as process aid or to achieve target pH | | |
| 25 | Citric Acid Anhydrous, supplier: Archer Daniels Midland; level adjustable to achieve target pH | | |
| 26 | Sodium Chloride, supplier: Morton; level adjustable to achieve target viscosity | | |

Gel Network with Fatty Acid Composition

| Ingredient | As added wt% |
|---|---|
| Water | 78.66% |
| Cetyl Alcohol | 4.06% |
| Steary Alcohol | 7.97% |
| Stearic acid | 8.23% |
| 50% NaOH Solution | 0.52% |

| Ingredients | Examples, active wt% | | |
|---|---|---|---|
| | 75 | 76 | 77 (comparative example) |
| Sodium Laureth-1 Sulfate (SLE1S) [1] | 7.0 | | |
| Sodium Undecyl Sulfate [2] | 4.0 | 8.5 | |
| Sodium Deceth-1 Sulfate [3] | | | 7.5 |
| Sodium Trideceth-2 Sulfate [4] | | | 7.5 |
| Sodium Lauroyl Sarcosinate [5] | | 8.5 | |
| Cocamidopropyl Betaine [6] | 2.0 | | |
| Cocamide MEA [7] | 1.5 | | |
| Piroctone Olamine [8] | 0.25 | 0.5 | 1.0 |
| Ammonium Acryloyldimethyl-taurate/VP Copolymer [9] | | 2.5 | |
| Polystyrene Sulfonate [9] | 1.0 | | 0.15 |
| Poly(methyl methacrylate/-methacrylic acid) [90:10 monomer ratio] [10] | | | 1.0 |
| Polyquaternium-10 [12] | | 0.3 | |
| Polyquaternium-10 [13] | 0.5 | | |
| Polyquaternium-67 [14] | | | 0.25 |
| Polyquaternium-76 [15] | | 0.1 | |
| Dimethicone [16] | 1.2 | | |
| Dimethicone [17a] | | 0.5 | |
| Glycerin [17b] | | 0.02 | |
| Trideceth-3 [17c] | | 0.02 | |
| Decyl Glucoside [17d] | | 0.1 | |
| Linoleamidopropyl PG-Dimonium Chloride Phosphate [18] | | | 0.7 |
| Glycerin [19] | 2.0 | | |
| Sodium Benzoate [20] | 0.25 | 0.25 | 0.25 |
| Tetrasodium EDTA [21] | 0.13 | 0.13 | 0.13 |
| Methylchloroisothiazolinone/ Methylisothiazolinone [22] | 5 ppm | 5 ppm | 5 ppm |
| Sodium Hydroxide [23] | Up to 1% | Up to 1% | Up to 1% |
| Citric Acid [24] | Up to 2% | Up to 2% | Up to 2% |
| Fragrance | 1.0 | 1.0 | 1.0 |
| Sodium Chloride [25] | Up to 3% | Up to 3% | Up to 3% |
| Water | q.s. | q.s. | q.s. |
| | | | |
| pH | 6.0 | 4.5 | 5.0 |
| 1 | Sodium Laureth-1 Sulfate at 26% active, supplier: P&G | | |
| 2 | Sodium Undecyl Suflate at 70% active, supplier: P&G | | |

(continued)

| Ingredients | Examples, active wt% | | |
|---|---|---|---|
| | 75 | 76 | 77 (comparative example) |
| 3 | Sodium Deceth-1 Sulfate at 70% active, supplier P&G | | |
| 4 | STEOL-TD 402-65 at 65% active, supplier: Stepan | | |
| 5 | Crodasinic LS-30NP at 30% active, supplier: Croda | | |
| 6 | Tego Betain L 7 OK at 30% active, supplier: Evonik | | |
| 7 | Ninol Comf at 85% active, supplier: Stepan | | |
| 8 | Octopirox, supplier: Clariant | | |
| 9 | Aristoflex AVC, supplier: Clariant | | |
| 10 | Polystyrene Sulfonate, supplier: Akzo | | |
| 11 | Poly(methyl methacrylate/methacrylic acid) [90:10 monomer ratio], supplier: Polysciences, Inc. | | |
| 12 | UCARE Polymer JR-400, supplier: Dow Chemical | | |
| 13 | Polyquaternium-10 with charge density = 2.6 meq/g and MW = 2MM available from Dow Chemical | | |
| 14 | SoftCAT Polymer SL-100, supplier: Dow Chemical | | |
| 15 | Mirapol AT-1 at 10% active, supplier: Solvay | | |
| 16 | CF330M, supplier: Momentive Dimethicone Emulsion having an average particle size of about 1.0 to about 10 microns. The dimethicone is emulsified separately using various emulsifiers and the completed emulsion is subsequently added to the composition. A non-limiting selection of emulsifiers (17b-d) are shown herein. | | |
| 17 | 17a: Element14 PDMS 100K, supplier: Momentive<br>17b: Glycerine 99.7% USP, supplier: P&G Chemicals<br>17c: Lutensol TDA 3, supplier: BASF Corporation<br>17d: Plantaren 2000 N UP, 50% active, supplier: BASF Corporation | | |
| 18 | Arlasilk EFA at 30% active, supplier: Croda | | |
| 19 | Glycerine 99.7% USP, supplier: P&G Chemicals | | |
| 20 | Sodium Benzoate Dense NF/FCC, supplier: Emerald Performance Materials | | |
| 21 | Dissolvine 220-S at 84% active, supplier: Akzo Nobel | | |
| 22 | Kathon CG at 1.5% active, supplier: Rohm & Haas | | |
| 23 | Sodium Hydroxide - Caustic Soda at 50% active, supplier: K.A. Steel Chemicals, Inc.; level adjustable as process aid or to achieve target pH | | |
| 24 | Citric Acid Anhydrous, supplier: Archer Daniels Midland; level adjustable to achieve target pH | | |
| 25 | Sodium Chloride, supplier: Morton; level adjustable to achieve target viscosity | | |

| Ingredients | Examples, active wt% | | |
|---|---|---|---|
| | 78 | 79 (comparative example) | 80 (comparative example) |
| Sodium Laureth-1 Sulfate (SLE1S) [1] | | | 12 |
| Sodium Deceth-1 Sulfate [2] | 7.5 | 7.5 | |

(continued)

| Ingredients | Examples, active wt% | | | |
|---|---|---|---|---|
| | 78 | 79 (comparative example) | 80 (comparative example) | |
| Sodium Decyl Sulfate 3 | | | 12 | |
| Sodium Trideceth-2 Sulfate [4] | 7.5 | 7.5 | | |
| Cocamide MEA [5] | | 0.5 | | |
| Piroctone Olamine [6] | 0.5 | 1.0 | 1.0 | |
| Polystyrene Sulfonate [7] | | 0.2 | 0.3 | |
| Sodium Carboxymethylcellu-lose [8] | 1.5 | | 0.5 | |
| Poly(methyl methacrylate/-methacrylic acid) [90:10 monomer ratio] [9] | 0.5 | | | |
| Cationic Starch [10] | 0.4 | | | |
| Polyquaternium-67 [11] | | 0.3 | | |
| Polyquaternium-24 [12] | | | 1.5 | |
| Gel Network [13] | | 14.0 | | |
| Dimethicone [14] | | 0.8 | | |
| Dimethiconol [15] | | | 1.5 | |
| Glycerin [16] | 1.0 | | | |
| Sodium Benzoate [17] | 0.5 | 0.5 | 0.25 | |
| Tetrasodium EDTA [18] | 0.13 | 0.13 | 0.13 | |
| Methylchloroisothiazolinone/ Methylisothiazolinone [19] | | | 5 ppm | |
| Sodium Salicylate [20] | | 0.02 | 0.02 | |
| Sodium Hydroxide [21] | | Up to 1% | Up to 1% | Up to 1% |
| Citric Acid [22] | | Up to 2% | Up to 2% | Up to 2% |
| Fragrance | | 1.0 | 1.0 | 1.0 |
| Sodium Chloride [23] | | Up to 3% | Up to 3% | Up to 3% |
| Water | | q.s. | q.s. | q.s. |
| | | | | |
| pH | | 7.0 | 6.0 | 4.0 |

| | |
|---|---|
| 1 | Sodium Laureth-1 Sulfate at 26% active, supplier: P&G |
| 2 | Sodium Deceth-1 Sulfate at 70% active, supplier P&G |
| 3 | Sodium Decyl Sulfate at 70% active, supplier P&G |
| 4 | STEOL-TD 402-65 at 65% active, supplier: Stepan |
| 5 | Ninol Comf at 85% active, supplier: Stepan |
| 6 | Octopirox, supplier: Clariant |
| 7 | Polystyrene Sulfonate, supplier: Akzo |
| 8 | Sodium Carboxymethylcellulose, supplier: Dow Chemical |
| 9 | Poly(methyl methacrylate/methacrylic acid) [90:10 monomer ratio], supplier: Polysciences, Inc. |

(continued)

| Ingredients | Examples, active wt% | | |
|---|---|---|---|
| | 78 | 79 (comparative example) | 80 (comparative example) |
| 10 | Cationic Starch, MW = 10,000,000, 3% N, supplier: National Starch | | |
| 11 | SoftCAT Polymer SK-H, supplier: Dow Chemical | | |
| 12 | Crodacel QS, supplier: Croda | | |
| 13 | Gel Network with Fatty Alcohol; See Composition in table below. The water is heated to about 74°C and the Cetyl Alcohol, Stearyl Alcohol, and the SLES Surfactant are added to it. After incorporation, this mixture is passed through a heat exchanger where it is cooled to about 35°C. As a result of this cooling step, the Fatty Alcohols and surfactant crystallize to form a crystalline gel network. | | |
| 14 | CF330M, supplier: Momentive | | |
| 15 | DC-1872 at 25% active, supplier: Dow Corning | | |
| 16 | Glycerine 99.7% USP, supplier: P&G Chemicals | | |
| 17 | Sodium Benzoate Dense NF/FCC, supplier: Emerald Performance Materials | | |
| 18 | Dissolvine 220-S at 84% active, supplier: Akzo Nobel | | |
| 19 | Kathon CG at 1.5% active, supplier: Rohm & Haas | | |
| 20 | Sodium Salicylate, supplier: JQC (Huayin) Pharmaceutical Co., Ltd. | | |
| 21 | Sodium Hydroxide - Caustic Soda at 50% active, supplier: K.A. Steel Chemicals, Inc.; level adjustable as process aid or to achieve target pH | | |
| 22 | Citric Acid Anhydrous, supplier: Archer Daniels Midland; level adjustable to achieve target pH | | |
| 23 | Sodium Chloride, supplier: Morton; level adjustable to achieve target viscosity | | |

Gel Network with Fatty Alcohol Composition

| Ingredient | As added wt% |
|---|---|
| Water | 45.66% |
| Cetyl Alcohol | 4.29% |
| Steary Alcohol | 7.71% |
| Sodium laureth-1 sulfate (26% Active) | 42.31% 5 |

(continued)

| Ingredient | As added wt% |
|---|---|
| Methylchloroisothiazolinone/ Methylisothiazolinone, Kathon CG | 0.03% |

## Claims

**1.** A hair care composition comprising:

a) from 10% to 25% of one or more surfactants, by weight of the composition, wherein the one or more surfactants comprises an anionic surfactant selected from the group consisting of anionic alkyl sulfates and alkyl ether sulfates having straight or branched alkyl chains and mixtures thereof;
b) from 0.01% to 10% of one or more surfactant soluble antidandruff agents, by weight of the composition, wherein the one or more surfactant soluble antidandruff agents comprises one material or a mixture selected from the groups consisting of: azoles being climbazole, ketoconazole, itraconazole, econazole, and elubiol; hydroxy pyridones being piroctone olamine, ciclopirox, rilopirox, and MEA-Hydroxyoctyloxypyridinone; kerolytic agent being salicylic acid; strobilurin being azoxystrobin; and metal chelator being 1,10-phenanthroline;
c) from 1% to 2.5% of one or more anionic polymers selected from the group consisting of anionic polymers with anionic functional groups, anionic polymers which are homopolymers having a monomer, or copolymers having more than one type of monomer including one or more monomers with anionic functional groups and other monomers that contain non anionic functional groups and mixtures thereof, by weight of the composition;
d) from 0.2% to 1% of one or more cationic polymers, by weight of the composition; wherein the composition has a deposition efficiency of greater than 1.2X that of a control composition, as measured according to the Anti-dandruff Agent Deposition Test Method as disclosed herein, wherein the control composition comprises 14% SLE1S by weight of the composition, no polymeric constituents, 1% of the surfactant soluble antidandruff agent by weight of the composition at a pH of 6, preferably wherein the composition has a deposition efficiency of greater than or equal to 1.4X that of a control composition wherein the control composition comprises 14% SLE1S by weight of the composition, 1% of the surfactant soluble antidandruff agent by weight of the composition at a pH of 6.

**2.** A hair care composition according to Claim 1, wherein the one or more cationic polymers are selected from the group consisting of a cationic guar polymer, a cationic non-guar galactomannan polymer, a cationic tapioca polymer, a cationic copolymer of acrylamide monomers and cationic monomers, a synthetic, non-crosslinked, cationic polymer, which may or may not form lyotropic liquid crystals upon combination with the detersive surfactant, a cationic cellulose polymer and mixtures thereof, preferably wherein the one or more cationic polymer is selected from the group consisting of guar hydroxypropyltrimonium chloride, salts of hydroxyethyl cellulose reacted with trimethyl ammonium substituted epoxide, a cationic copolymer of acrylamide monomers and cationic monomers, a synthetic, non-crosslinked, cationic polymer, which may or may not form lyotropic liquid crystals upon combination with the detersive surfactant.

**3.** A hair care composition according to any preceding claims, wherein the one or more anionic functional groups of the one or more anionic polymers is selected from the group consisting a polyacrylate, polyacrylamide polymer, anionic polymers that are homopolymers based on acrylic acid, methacrylic acid or other related derivatives, alkali swellable and hydrophobically-modified alkali swellable acrylic copolymers or methacrylate copolymers, soluble crosslinked acrylic polymers, associative polymers and mixtures thereof.

**4.** A hair care composition according to any preceding claims, wherein the surfactant is an anionic surfactant selected from the group consisting of:

a) $R_1\ O(CH_2CHR_3O)_y\ SO_3M$;
b) $CH_3\ (CH_2)_z\ CHR_2\ CH_2\ O\ (CH_2\ CHR_3O)_y\ SO_3M$; and
c) mixtures thereof,
where $R_1$ represents $CH_3\ (CH_2)_{10}$, $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is 1 when y is not zero (0), and M is a monovalent or divalent, positively-charged cation, preferably wherein the surfactant is a surfactant or combination of surfactants selected from the group consisting of sodium lauryl sulfate, sodium

laureth-n sulfate where n is between 0.5 to 3.5, sodium C10-15 alkyl sulfate where the alkyl chain can be linear or branched, sodium C10-15 pareth-n sulfate where n is between 0.5 to 3.5 and the alkyl chain can be linear or branched, sodium decyl sulfate, sodium deceth-n sulfate where n is between 0.5 to 3.5, sodium undecyl sulfate, sodium undeceth-n sulfate where n is between 0.5 to 3.5, sodium tridecyl sulfate, sodium trideceth-n sulfate where n is between 0.5 to 3.5, an anionic surfactant selected from the group consisting of:

a) R1 O(CH2CHR3O)y SO3M;
b) CH3 (CH2)z CHR2 CH2 O (CH2 CHR3O)y SO3M; and
c) mixtures thereof,

where R1 represents CH3 (CH2)10, R2 represents H or a hydrocarbon radical comprising 1 to 4 carbon atoms such that the sum of the carbon atoms in z and R2 is 8, R3 is H or CH3, y is 0 to 7, the average value of y is 1 when y is not zero (0), and M is a monovalent or divalent, positively-charged cation.

**5.** A hair care composition according to any preceding claims wherein one or more surfactants is from 10% to 18% by weight of the composition, preferably from 10% to 14% by weight of the composition, preferably from 10% to 12% by weight of the composition.

**6.** A hair care composition according to any preceding claims further comprising from 0.25% to 15% of one or more amphoteric, nonionic or zwitterionic co-surfactants, by weight of the composition.

**7.** A hair care composition according to any preceding claims wherein the pH of the composition is from 4 to 9, preferably from 4 to 6, preferably is from 4 to 5.5, preferably from 4 to 5.

**8.** A hair care composition according to any preceding claims wherein the surfactant soluble antidandruff agent is piroctone olamine.

**9.** A hair care composition according to any one of the claims 1-7 wherein the surfactant soluble antidandruff agent is climbazole.

**10.** A hair care composition according to any preceding claims further comprising from 0.1% to 9% by weight of the composition, preferably 0.25% to 8 by weight of the composition, of one or more scalp health agents selected from the group consisting of pyridinethione salts, selenium sulfide, particulate sulfur, menthol, menthyl lactate and mixtures thereof, preferably wherein the one or more scalp health agents is polyvalent metal salts of pyrithione, preferably wherein the one or more scalp health agents is zinc pyrithione.

**11.** A hair care composition according to any preceding claims wherein the composition further comprising a conditioning agent, preferably wherein the conditioning agent is a silicone.

**12.** A hair care composition according to any preceding claims wherein the hair care composition is dispensed as a foam, preferably wherein the hair care composition is dispensed as an aerosol foam, preferably wherein the hair care composition is dispensed as a pumped foam.

## Patentansprüche

**1.** Haarpflegezusammensetzung, umfassend:

a) zu von 10 Gew.-% bis 25 Gew.-% der Zusammensetzung ein oder mehrere Tenside, wobei das eine oder die mehreren Tenside ein anionisches Tensid umfassen, ausgewählt aus der Gruppe bestehend aus anionischen Alkylsulfaten und Alkylethersulfaten, die gerade oder verzweigte Alkylketten und Mischungen davon aufweisen;
b) zu von 0,01 Gew.-% bis 10 Gew.-% der Zusammensetzung ein oder mehrere tensidlösliche Antischuppenmittel, wobei das eine oder die mehreren tensidlöslichen Antischuppenmittel ein Material oder eine Mischung umfassen, ausgewählt aus den Gruppen bestehend aus: Azolen, die Climbazol, Ketoconazol, Itraconazol, Econazol und Elubiol sind; Hydroxypyridonen, die Piroctonolamin, Ciclopirox, Rilopirox oder MEA-Hydroxyoctyloxyypyridinon sind; einem keratolytischen Mittel, das Salicylsäure ist; Strobilurin, das Azoxystrobin ist; und dem Metallchelator, der 1,10-Phenanthrolin ist;
c) zu von 1 Gew-% bis 2,5 Gew.-% der Zusammensetzung ein oder mehrere anionische Polymere, ausgewählt

aus der Gruppe bestehend aus anionischen Polymeren mit anionischen Funktionsgruppen, anionischen Polymeren, die Homopolymere sind, die ein Monomer aufweisen, oder Copolymeren, die mehr als eine Art von Monomer aufweisen, einschließlich einem oder mehreren Monomeren mit anionischen Funktionsgruppen und anderen Monomeren, die nicht anionische Funktionsgruppen enthalten, und Mischungen davon;
d) zu von 0,2 Gew.-% bis 1 Gew.-% der Zusammensetzung ein oder mehrere kationische Polymere;
wobei die Zusammensetzung eine Ablagerungseffizienz von mehr als dem 1,2-fachen einer Steuerzusammensetzung aufweist, gemessen gemäß dem hierin offenbarten Prüfverfahren für die Ablagerung von Antischuppenmitteln, wobei die Steuerzusammensetzung zu 14 Gew.-% der Zusammensetzung SLE1S, keine polymeren Bestandteile, zu 1 Gew.-% der Zusammensetzung das tensidlösliche Antischuppenmittel bei einem pH-Wert von 6 umfasst, wobei die Zusammensetzung vorzugsweise eine Ablagerungseffizienz von mehr als oder gleich dem 1,4-fachen einer Steuerzusammensetzung aufweist, wobei die Steuerzusammensetzung zu 14 Gew.-% der Zusammensetzung SLE1S und zu 1 Gew.-% der Zusammensetzung das tensidlösliche Antischuppenmittel bei einem pH-Wert von 6 umfasst.

**2.** Haarpflegezusammensetzung nach Anspruch 1, wobei das eine oder die mehreren kationischen Polymere aus der Gruppe ausgewählt sind, bestehend aus einem kationischen Guarpolymer, einem kationischen Nicht-Guargalactomannanpolymer, einem kationischen Tapiokapolymer, einem kationischen Copolymer von Acrylamidmonomeren und kationischen Monomeren, einem synthetischen, nicht vernetzten kationischen Polymer, das bei Kombination mit dem Reinigungstensid lyotrope Flüssigkristalle ausbildet oder nicht, einem kationischen Cellulosepolymer und Mischungen davon, vorzugsweise wobei das eine oder die mehreren kationischen Polymere ausgewählt sind aus der Gruppe bestehend aus Guarhydroxypropyltrimoniumchlorid, Salzen von Hydroxyethylcellulose, die mit Trimethylammonium-substituiertem Epoxid reagiert sind, einem kationischen Copolymer von Acrylamidmonomeren und kationischen Monomeren, einem synthetischen, nicht vernetzten kationischen Polymer, das bei Kombination mit dem Reinigungstensid lyotrope Flüssigkristalle ausbildet oder nicht.

**3.** Haarpflegezusammensetzung nach einem der vorstehenden Ansprüche, wobei die eine oder die mehreren anionischen Funktionsgruppen der einen oder der mehreren anionischen Polymere ausgewählt sind aus der Gruppe bestehend aus einem Polyacrylat, einem Polyacrylamidpolymer, anionischen Polymeren, die Homopolymere auf Basis von Acrylsäure, Methacrylsäure oder anderen verwandten Derivaten sind, alkaliquellbaren und hydrophob modifizierten alkaliquellbaren Acrylcopolymeren oder Methacrylatcopolymeren, löslichen vernetzten Acrylpolymeren, assoziativen Polymeren und Mischungen davon.

**4.** Haarpflegezusammensetzung nach einem der vorstehenden Ansprüche, wobei das Tensid ein anionisches Tensid ist, das ausgewählt ist aus der Gruppe bestehend aus:

a) $R_1$ $O(CH_2CHR_3O)_y$ $SO_3M$;
b) $CH_3$ $(CH_2)_z$ $CHR_2$ $CH_2$ O $(CH_2$ $CHR_3O)_y$ $SO_3M$; und
c) Mischungen davon,

wobei $R_1$ $CH_3$ $(CH_2)_{10}$ darstellt, $R_2$ H oder ein Kohlenwasserstoffradikal, umfassend 1 bis 4 Kohlenstoffatome, derart darstellt, dass die Summe der Kohlenstoffatome in z und $R_2$ 8 beträgt, $R_3$ H oder $CH_3$ ist, y 0 bis 7 ist, der durchschnittliche Wert von y 1 ist, wenn y nicht null (0) ist, und M ein einwertiges oder zweiwertiges, positiv geladenes Kation ist, wobei das Tensid vorzugsweise ein Tensid oder eine Kombination von Tensiden ist, ausgewählt aus der Gruppe bestehend aus Natriumlaurylsulfat, Natriumlaureth-n-Sulfat, wobei n zwischen 0,5 und 3,5 liegt, Natrium-C10-15-Alkylsulfat, wobei die Alkylkette linear oder verzweigt sein kann, Natrium-C10-15-Pareth-n-Sulfat, wobei n zwischen 0,5 und 3,5 liegt und die Alkylkette linear oder verzweigt sein kann, Natriumdecylsulfat, Natriumdeceth-n-Sulfat, wobei n zwischen 0,5 und 3,5 liegt, Natriumundecylsulfat, Natriumundeceth-n-Sulfat, wobei n zwischen 0,5 und 3,5 liegt, Natriumtridecylsulfat, Natriumtrideceth-n-Sulfat, wobei n zwischen 0,5 und 3,5 liegt, einem anionischen Tensid, ausgewählt aus der Gruppe bestehend aus:

a) R1 O(CH2CHR3O)y SO3M;
b) CH3 (CH2)z CHR2 CH2 O (CH2 CHR3O)y SO3M; und
c) Mischungen davon,

wobei R1 CH3 (CH2)10 darstellt, R2 H oder ein Kohlenwasserstoffradikal, umfassend 1 bis 4 Kohlenstoffatome, derart darstellt, dass die Summe der Kohlenstoffatome in z und R2 8 beträgt, R3 H oder CH3 ist, y 0 bis 7 ist, der Durchschnittswert von y 1 ist, wenn y nicht null (0) ist, und M ein einwertiges oder zweiwertiges,

positiv geladenes Kation ist.

5. Haarpflegezusammensetzung nach einem der vorstehenden Ansprüche, wobei ein oder mehrere Tenside zu 10 Gew.-% bis 18 Gew.-% der Zusammensetzung, vorzugsweise zu 10 Gew.-% bis 14 Gew.-% der Zusammensetzung und vorzugsweise zu 10 Gew.-% bis 12 Gew.-% der Zusammensetzung vorliegen.

6. Haarpflegezusammensetzung nach einem der vorstehenden Ansprüche, ferner umfassend zu 0,25 Gew.-% bis 15 Gew.-% der Zusammensetzung ein oder mehrere amphotere, nichtionische oder zwitterionische Cotenside.

7. Haarpflegezusammensetzung nach einem der vorstehenden Ansprüche, wobei der pH-Wert der Zusammensetzung von 4 bis 9, vorzugsweise von 4 bis 6, vorzugsweise von 4 bis 5,5, vorzugsweise von 4 bis 5 beträgt.

8. Haarpflegezusammensetzung nach einem der vorstehenden Ansprüche, wobei das tensidlösliche Antischuppenmittel Piroctonolamin ist.

9. Haarpflegezusammensetzung nach einem der Ansprüche 1 bis 7, wobei das tensidlösliche Antischuppenmittel Climbazol ist.

10. Haarpflegezusammensetzung nach einem der vorstehenden Ansprüche, ferner umfassend zu 0,1 bis 9 Gew.-% der Zusammensetzung, vorzugsweise zu 0,25 bis 8 Gew.-% der Zusammensetzung, ein oder mehrere Kopfhautgesundheitsmittel, ausgewählt aus der Gruppe bestehend aus Pyridinthionsalzen, Selensulfid, partikulärem Schwefel, Menthol, Menthyllactat und Mischungen davon, wobei das eine oder die mehreren Kopfhautgesundheitsmittel vorzugsweise mehrwertige Metallsalze von Pyrithion sind, wobei das eine oder die mehreren Kopfhautgesundheitsmittel vorzugsweise Zinkpyrithion sind.

11. Haarpflegezusammensetzung nach einem der vorstehenden Ansprüche, wobei die Zusammensetzung ferner ein Konditioniermittel umfasst, wobei das Konditioniermittel vorzugsweise ein Silikon ist.

12. Haarpflegezusammensetzung nach einem der vorstehenden Ansprüche, wobei die Haarpflegezusammensetzung als ein Schaum abgegeben wird, wobei die Haarpflegezusammensetzung vorzugsweise als ein Aerosolschaum abgegeben wird, wobei die Haarpflegezusammensetzung vorzugsweise als ein gepumpter Schaum abgegeben wird.

## Revendications

1. Composition pour soins capillaires comprenant :

a) de 10 % à 25 % d'un ou plusieurs agents tensioactifs, en poids de la composition, dans laquelle le ou les agents tensioactifs comprennent un agent tensioactif anionique choisi dans le groupe constitué de sulfates d'alkyle anioniques et sulfates d'éther alkylique ayant des chaînes alkyle linéaires ou ramifiées et mélanges de ceux-ci ;
b) de 0,01 % à 10 % d'un ou plusieurs agents antipelliculaires solubles dans un agent tensioactif, en poids de la composition, dans laquelle le ou les agents antipelliculaires solubles dans un agent tensioactif comprennent un matériau ou un mélange choisi parmi les groupes constitués par : des azoles étant climbazole, kétoconazole, itraconazole, éconazole, ou élubiol ; des hydroxy pyridones étant piroctone olamine, ciclopirox, rilopirox, et MEA-hydroxyoctyloxypyridinone ; un agent kérolytique étant un acide salicylique ; une strobilurine étant azoxystrobine ; et le chélateur de métaux étant 1,10-phénanthroline ;
c) de 1 % à 2,5 % d'un ou plusieurs polymères anioniques choisis dans le groupe constitué de polymères anioniques avec des groupes fonctionnels anioniques, polymères anioniques qui sont des homopolymères ayant un monomère, ou copolymères ayant plus d'un type de monomère comportant un ou plusieurs monomères avec des groupes fonctionnels anioniques et d'autres monomères qui contiennent des groupes fonctionnels non anioniques et des mélanges de ceux-ci, en poids de la composition ;
d) de 0,2 % à 1 % d'un ou plusieurs polymères cationiques, en poids de la composition ;
dans laquelle la composition a une efficacité de dépôt supérieure à 1,2X celle d'une composition témoin, telle que mesurée selon le procédé d'essai de dépôt d'agent antipelliculaire tel que divulgué ici, dans laquelle la composition témoin comprend 14 % de SLE1S en poids de la composition, aucun constituant polymère, 1 % de l'agent antipelliculaire soluble dans un agent tensioactif en poids de la composition à un pH de 6, de préférence dans laquelle la composition a une efficacité de dépôt supérieure ou égale à 1.4X celle d'une composition témoin,

dans laquelle la composition témoin comprend 14 % de SLE1S en poids de la composition, 1 % d'agent antipelliculaire soluble dans un agent tensioactif en poids de la composition à un pH de 6.

2. Composition pour soins capillaires selon la revendication 1, dans laquelle le ou les polymères cationiques sont choisis dans le groupe constitué d'un polymère de guar cationique, un polymère galactomannane non de guar cationique, un polymère de tapioca cationique, un copolymère cationique de monomères acrylamides et de monomères cationiques, un polymère cationique, synthétique, non réticulé, qui peut ou non former des cristaux liquides lyotropes lors de la combinaison avec l'agent tensioactif détersif, un polymère de cellulose cationique et des mélanges de ceux-ci, de préférence dans laquelle le ou les polymères cationiques sont choisis dans le groupe constitué de chlorure d'hydroxypropyltrimonium de guar, de sels d'hydroxyéthylcellulose mis à réagir avec un époxyde substitué par triméthylammonium, de copolymère cationique de monomères acrylamides et de monomères cationiques, de polymère cationique, synthétique, non réticulé, qui peut ou non former des cristaux liquides lyotropes lors de la combinaison avec l'agent tensioactif détersif.

3. Composition pour soins capillaires selon l'une quelconque des revendications précédentes, dans laquelle le ou les groupes fonctionnels anioniques du ou des polymères anioniques sont choisis dans le groupe constitué d'un polyacrylate, d'un polymère de polyacrylamide, de polymères anioniques qui sont des homopolymères à base d'acide acrylique, d'acide méthacrylique ou d'autres dérivés apparentés, de copolymères acryliques ou de copolymères de méthacrylate gonflant en milieu alcalin et gonflant en milieu alcalin rendus hydrophobes, de polymères acryliques réticulés solubles, de polymères associatifs et de mélanges de ceux-ci.

4. Composition pour soins capillaires selon l'une quelconque des revendications précédentes, dans laquelle l'agent tensioactif est un agent tensioactif anionique choisi dans le groupe constitué de :

a) $R_1$ $O(CH_2CHR_3O)_y$ $SO_3M$ ;
b) $CH_3$ $(CH_2)_z$ $CHR_2$ $CH_2$ O $(CH_2$ $CHR_3O)_y$ $SO_3M$ ; et
c) mélanges de ceux-ci,

où $R_1$ représente $CH_3$ $(CH_2)_{10}$, $R_2$ représente H ou un radical hydrocarboné comprenant 1 à 4 atomes de carbone de telle sorte que la somme des atomes de carbone dans z et $R_2$ vaut 8, $R_3$ est H ou $CH_3$, y va de 0 à 7, la valeur moyenne de y vaut 1 lorsque y n'est pas zéro (0), et M est un cation chargé positivement, monovalent ou divalent, de préférence dans laquelle l'agent tensioactif est un agent tensioactif ou une combinaison d'agents tensioactifs choisi dans le groupe constitué de laurylsulfate de sodium, laureth-n sulfate de sodium où n est compris entre 0,5 et 3,5, alkylsulfate en C10 à 15 de sodium où la chaîne alkyle peut être linéaire ou ramifiée, pareth-n sulfate de sodium en C10 à 15 où n est compris entre 0,5 et 3,5 et la chaîne alkyle peut être linéaire ou ramifiée, décylsulfate de sodium, déceth-n sulfate de sodium où n est compris entre 0,5 et 3,5, undécyl-sulfate de sodium, undéceth-n sulfate de sodium où n est compris entre 0,5 et 3,5, tridécyl-sulfate de sodium, tridéceth-n sulfate de sodium où n est compris entre 0,5 et 3,5, un agent tensioactif anionique choisi dans le groupe constitué de :

a) R1 O(CH2CHR3O)y SO3M ;
b) CH3 (CH2)z CHR2 CH2 O (CH2 CHR3O)y SO3M ; et
c) mélanges de ceux-ci,

où R1 représente CH3 (CH2)10, R2 représente H ou un radical hydrocarboné comprenant 1 à 4 atomes de carbone de telle sorte que la somme des atomes de carbone dans z et R2 vaut 8, R3 est H ou CH3, y va de 0 à 7, la valeur moyenne de y vaut 1 lorsque y n'est pas zéro (0), et M est un cation chargé positivement, monovalent ou divalent.

5. Composition pour soins capillaires selon l'une quelconque des revendications précédentes, dans laquelle un ou plusieurs agents tensioactifs vont de 10 % à 18 % en poids de la composition, de préférence de 10 % à 14 % en poids de la composition, de préférence de 10 % à 12 % en poids de la composition.

6. Composition pour soins capillaires selon l'une quelconque des revendications précédentes, comprenant en outre de 0,25 % à 15 % d'un ou plusieurs agents co-tensioactifs amphotères, non ioniques ou zwittérioniques, en poids de la composition.

7. Composition pour soins capillaires selon l'une quelconque des revendications précédentes, dans laquelle le pH de la

composition va de 4 à 9, de préférence de 4 à 6, de préférence de 4 à 5,5, de préférence de 4 à 5.

**8.** Composition pour soins capillaires selon l'une quelconque des revendications précédentes, dans laquelle l'agent antipelliculaire soluble dans un agent tensioactif est la piroctone olamine.

**9.** Composition pour soins capillaires selon l'une quelconque des revendications 1 à 7, dans laquelle l'agent antipelliculaire soluble dans un agent tensioactif est le climbazole.

**10.** Composition pour soins capillaires selon l'une quelconque des revendications précédentes, comprenant en outre de 0,1 % à 9 % en poids de la composition, de préférence de 0,25 % à 8 % en poids de la composition, d'un ou plusieurs agents de santé du cuir chevelu choisis dans le groupe constitué de sels de pyridinethione, sulfure de sélénium, soufre particulaire, menthol, lactate de menthyle et de mélanges de ceux-ci, de préférence dans laquelle le ou les agents de santé du cuir chevelu sont des sels métalliques polyvalents de pyrithione, de préférence dans laquelle le ou les agents de santé du cuir chevelu sont la pyrithione de zinc.

**11.** Composition pour soins capillaires selon l'une quelconque des revendications précédentes, dans laquelle la composition comprend en outre un agent de conditionnement, de préférence dans laquelle l'agent de conditionnement est une silicone.

**12.** Composition pour soins capillaires selon l'une quelconque des revendications précédentes, dans laquelle la composition pour soins capillaires est distribuée sous forme de mousse, de préférence dans laquelle la composition pour soins capillaires est distribuée sous forme de mousse aérosol, de préférence dans laquelle la composition pour soins capillaires est distribuée sous forme de mousse pompée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016172409 A1
- US 8440605 B
- US 2009155383
- US 20090221463
- US 2486921 A
- US 2486922 A
- US 2396278 A
- US 5104646 A
- US 5106609 A
- US 3929678 A
- US 2658072 A
- US 2438091 A
- US 2528378 A
- WO 9406403 A, Reich
- US 34584 A
- US 6316541 B
- US 4476282 A
- US 20070276087
- US 9255184 B2
- US 7683119 B
- US 5284972 A
- US 5747440 A
- WO 2004078903 A
- WO 2004078901 A
- WO 2005078063 A

### Non-patent literature cited in the description

- **PATIST, A.** ; **JHA, B.K.** ; **OH, S.G.** ; **SHAH, D.O.** *J. Surfactants Deterg.*, 1999, vol. 2, 317
- **JAMES-SMITH, M.A.** ; **SHEKHAWAT, D.** ; **SHAH, D.O.** *Tenside Surf. Det.*, 2007, vol. 44, 142
- McCutcheion's Detergents and Emulsifiers. Allured Publishing Corp., 1986
- McCutcheion's Functional Materials. 1992
- McCutcheon's, Emulsifiers and Detergents. M. C. Publishing Co., 1989
- **SOLAREK, D. B.** Cationic Starches in Modified Starches: Properties and Uses. CRC Press, Inc., 1986, 113-125
- **QIN-JI PENG** ; **ARTHUR S. PERLIN**. Observation on NMR Spectra of Starches in Dimethyl Sulfoxide, Iodine-Complexing, and Solvating in Water-Dimethyl Sulfoxide. *Carbohydrate Research*, 1987, vol. 160, 57-72
- **J. HOWARD BRADBURY** ; **J. GRANT COLLINS**. An Approach to the Structural Analysis of Oligosaccharides by NMR Spectroscopy. *Carbohydrate Research*, 1979, vol. 71, 15-25
- The CTFA Cosmetic Ingredient Handbook. Cosmetic, Toiletry, and Fragrance Association, Inc., 2004
- Encyclopedia of Polymer Science and Engineering. John Wiley & Sons, Inc., 1989, vol. 15, 204-308
- Emulsions and Emulsion Stability. CRC Press, 2005
- **SMITH, A. L.** The Analytical Chemistry of Silicones. John Wiley & Sons, Inc., 1991
- **A E MARTELL** ; **R M SMITH**. Critical Stability Constants. Plenum Press, 1974, vol. 1
- **A E MARTELL** ; **R D HANCOCK**. Metal Complexes in Aqueous Solution. Plenum Press, 1996